# EUROPEAN PATENT APPLICATION

(11) **EP 4 765 129 A1**
(43) Date of publication of application: **24.06.2026**
(21) Application number: 24222261.0
(22) Date of filing: 20.12.2024
(51) Int. Cl.: G16B 5/00

(54) **METHOD FOR THE DETERMINATION OF OXIDATIVE PHOSPHORYLATION PROFILES**

(71) Applicant: Doppelganger Biosystem GmbH, 26121 Oldenburg (DE)
(72) Inventor: DETMERS, Jan, 26939 Ovelgönne (DE)
(74) Representative: Maiwald GmbH

(57) **Abstract**

The present invention relates to a computer-implemented method for determining the oxidative phosphorylation profile of a cell sample, the method comprising a) determining the expression level(s) of adenine nucleotide translocator (ANT) and optionally one or more further targets involved in the oxidative metabolic phosphorylation pathway in the cell sample; b) providing the expression level data obtained in step a) to a mathematical model for metabolic profiling; and c) determining the oxidative phosphorylation profile of the cell sample (representative of its mitochondrial respiration profile) by calculation. Furthermore, the invention is directed to a computer program product configured to execute the computer-implemented method according to the invention on a computer.

## Description

### TECHNICAL FIELD

The present invention relates to a computer-implemented method for determining the oxidative phosphorylation profile of a cell sample, the method comprising a) determining the expression level(s) of adenine nucleotide translocator (ANT) and optionally one or more further targets involved in the oxidative metabolic phosphorylation pathway in the cell sample; b) providing the expression level data obtained in step a) to a mathematical model for metabolic profiling; and c) determining/calculating the oxidative phosphorylation profile of the cell sample (representative of its mitochondrial respiration profile). Furthermore, the invention is directed to a computer program product configured to execute the computer-implemented method according to the invention on a computer.

### BACKGROUND OF THE INVENTION

Oxidative phosphorylation (OXPHOS) is the central biological process responsible for energy production (ATP generation). The required energy is produced via the respiratory chain (Complexes 1-4) and converted into chemical energy through chemiosmotic coupling (Complex 5). Additionally, a transport protein (complex 6; adenine nucleotide translocator; ANT) embedded in the inner mitochondrial membrane transports ADP from the cytosol into the mitochondria and exports ATP from the mitochondria to the cytosol. This exchange ensures a continuous supply of ADP to the mitochondria for ATP production and delivers newly synthesized ATP to the cytosol, where it is used by the cell.

The cellular energy metabolism including the oxidative phosphorylation (OXPHOS) is a ubiquitous central biomarker in eukaryotes for cellular pathogenesis and therapy, making it scientifically and economically relevant.

Quantifying the oxidative phosphorylation potential of a subject has been found to be useful in a variety of applications and fields such as in metabolic research, oncology, neuroscience, cardiovascular research, stem cell research, aging research, immunology, infection biology, pharmacokinetics, toxicology, nutrition science, sports science, cancer immunotherapy, mitochondrial research, transplantation medicine, virology, biotechnology, microbiology, environmental research, drug discovery, development, precision therapy, drug safety, combination therapy, diagnostics, cell therapy, monitoring, and epidemiology.

There is thus need in the art for methods that allow determining the oxidative phosphorylation potential of a subject.

To date, there are a variety of techniques known in the art that can be used to quantify or infer ATP production rates. Some of these techniques include:
- Luciferase-Based Assays (ATP Bioluminescence Assay) - Measures ATP levels using a luciferase enzyme that emits light proportional to the ATP concentration in the sample;
- Seahorse XF Analyzer (Extracellular Flux Analysis) - Measures the oxygen consumption rate (OCR) and extracellular acidification rate (ECAR) of cells to determine ATP production rates through oxidative phosphorylation and glycolysis;
- 13C NMR Spectroscopy - Tracks the incorporation of labeled carbon (13C) from substrates into ATP to measure the rate of ATP synthesis;
- HPLC (High-Performance Liquid Chromatography) - Separates and quantifies ATP and its metabolites in cell extracts to assess ATP production and consumption;
- Mass Spectrometry (MS) - Quantifies ATP and other nucleotides, allowing precise measurement of ATP levels and metabolic flux;
- Fluorescence-Based Assays - Uses ATP-sensitive fluorescent dyes or proteins to measure ATP concentration changes in real-time within cells or tissues;
- Phosphorescence Lifetime Imaging Microscopy (PLIM) - Measures the oxygen-dependent phosphorescence lifetime of specific probes to infer ATP production rates indirectly via oxygen consumption;
- Radioisotope Labeling (e.g., [32P] Phosphate) - Incorporates radioactive phosphate into ATP molecules to measure the rate of ATP synthesis in isolated mitochondria or cells;
- FRET-Based ATP Sensors (Fluorescence Resonance Energy Transfer) - Uses genetically encoded sensors that change fluorescence upon binding ATP, allowing for dynamic and real-time measurement of ATP levels in living cells;
- Oxygraph (Clark Electrode) - Measures oxygen consumption rates in isolated mitochondria or cells to estimate ATP production from oxidative phosphorylation;
- Colorimetric Assays (e.g., MTT or Resazurin Reduction Assay) - Indirectly estimates ATP production by assessing cell viability and metabolic activity;
- MALDI-TOF Mass Spectrometry - Analyzes ATP and its degradation products to monitor changes in ATP production rates;
- Respirometry (e.g., Oroboros O2k) - Measures oxygen consumption rates and mitochondrial function to infer ATP production efficiency;
- Bioenergetics Profiling - Combines multiple techniques (e.g., oxygen consumption, substrate utilization) to create a comprehensive profile of ATP production pathways;
- ADP/ATP Ratio Assays - Measures the ratio of ADP to ATP to infer changes in ATP production and consumption rates;
- ATP Synthase Activity Assay - Directly measures the enzymatic activity of ATP synthase to assess ATP production in mitochondria;
- Mitochondrial Membrane Potential Assays (e.g., JC-1 Dye) - Estimates ATP production by assessing changes in mitochondrial membrane potential, which correlates with ATP synthesis rates;
- Polarography - Measures changes in oxygen concentration in a closed system to assess mitochondrial respiration and ATP production;
- Oxygen Optode Systems - Utilizes optical sensors to measure oxygen concentration in real-time, providing insights into cellular respiration and ATP production rates;
- Calorimetry (Isothermal Microcalorimetry) - Measures the heat production rate of cells, which can be correlated with ATP production rates.

Despite the multitude of different methods known in the art for evaluating and quantifying oxidative phosphorylation rates, for example by means of ATP production rates, there exists need for further methods that avoid some of the drawbacks of existing methods, such as lack of single-cell analysis, lack of tissue analysis, expensive instrumentation, laborious procedures, lack of sensitivity and the like.

### SUMMARY OF THE INVENTION

The present invention is based on a method for determining a metabolic profile of a subject that uses a kinetic model comprising the major cellular metabolic pathways of cellular carbohydrate, lipid, ketone body- and amino acid metabolism as well as key electrophysiological processes at the inner mitochondrial membrane, including the membrane transport of various ions, the mitochondrial membrane potential and the generation and utilization of the proton-motive force. This method provides a robust approach to assessing the metabolic status of a subject, facilitating insights into energy metabolism and related physiological, pathological or therapeutic conditions. The model uses an algorithm that can quantify metabolic rates for up to 25 central metabolic pathways using up to 618 protein/transcript abundances.

The inventor surprisingly found that if quantifying the oxidative phosphorylation rates using this model, the results correlate surprisingly well with the results obtained for complex 6 (ANT) alone. This means that establishing an oxidative phosphorylation profile for a cell or subject based on determining the expression level(s) of adenine nucleotide translocator (ANT) alone yields a result that has an extremely high likelihood to be identical or highly similar to the oxidative phosphorylation profile established based on the determination of the expression level(s) of multiple or all of the components involved in the oxidative phosphorylation pathway (i.e. complexes 1-5, each consisting of more than one component). It is apparent that this finding significantly simplifies the determination of the oxidative phosphorylation profile of a cell, a tissue or a subject, since it requires determining the expression level of a single target, namely ANT, only without significantly compromising the validity of the result relative to the result obtained if determining the expression levels of multiple or all components of this metabolic pathway.

More specifically, a computer-implemented method for quantifying the oxidative phosphorylation potential of a subject has been developed by the inventor, the method comprising quantification of adenine nucleotide translocator (ANT) expression levels, preferably RNA or protein-based expression levels, and using the generated data as input for a mathematical model to determine the energy metabolic potential of the subject. The mathematical model can be parametrized using experimentally measured parameters and can simulate oxidative phosphorylation potential under various conditions.

Consequently, in a first aspect, the present invention relates to a computer-implemented method for determining the oxidative phosphorylation profile of a cell sample, the method comprising
a) determining the expression level(s) of adenine nucleotide translocator (ANT) and optionally one or more further targets involved in the oxidative metabolic phosphorylation pathway in the cell sample;
b) providing the expression level data obtained in step a) to a mathematical model for metabolic profiling; and
c) determining the oxidative phosphorylation profile of the cell sample (representative of its mitochondrial respiration profile) by calculation using the mathematical model.

In various embodiments, the cell sample is a single cell, cell suspension, organoid, membrane-surrounded particle or a tissue sample. Membrane-surrounded particles may include exosomes and extracellular vesicles. The tissue sample may be a tissue biopsy sample or a spatial tissue section.

In various embodiments, the oxidative phosphorylation profile is determined at single-cell level, for example to measure immune cell metabolism deficiency or brain cell metabolism abnormality. In various other embodiments, it is determined in bulk, for example biopsy, or spatial, for example pathology, scale.

In various embodiments, the cell sample is a mammalian cell sample, preferably a human cell sample.

In various embodiments, the cell sample has been obtained from a subject, preferably a mammal, more preferably a human subject.

In various embodiments, the expression level is determined by determining the total mRNA level and/or protein level of ANT variants, typically including all isoforms thereof, in the sample. While there exist 4 different isoforms of ANT in humans, these are differentially expressed in various tissues and cells. Depending on the tissue or cell type, the total mRNA level and/or protein level of one or more ANT isoforms, typically the prevalent ones in the respective tissue or cell, are determined. However, in various embodiments, the total mRNA level and/or protein level of all four ANT variants/isoforms are determined. Said determination does not need to differentiate between the different isoforms, but it is sufficient if the total mRNA level and/or protein level of all ANT variants in the sample is determined.

In various embodiments, the method further comprises determining the metabolic potential and/or the energy phenotype of the cell sample from the determined oxidative phosphorylation profile.

The method may further comprise the step of comparing the determined oxidative phosphorylation profile of the cell sample to a reference profile. Preferably, the reference profile is a healthy cell profile or a diseased cell profile. In various embodiments, the difference between the sample profile and the reference profile is
(a) indicative for a disease or disorder that affects the oxidative phosphorylation profile of a cell;
(b) used to determine susceptibility to a specific treatment of a disease or disorder;
(c) used for risk stratification to develop a disease or disorder;
(d) used to monitor the progression or treatment of a disease or disorder;
(e) used to screen potential pharmaceutical actives for their pharmaceutical activity, safety, and/or metabolism;
(f) used to determine the age, nutritional status and/or overall health of a subject; and/or
(g) used to determine the inflammation status, infection status, hereditary disease status, epidemiologic status, environmental harm, or intoxication status of a subject.

In the above embodiments, the disease or disorder may be selected from the group of neurodegenerative diseases or disorders, proliferative diseases or disorders, infectious diseases, oncologic diseases, mental disorders, cardiologic diseases or disorders, immune diseases or disorders, inflammation and metabolic diseases or disorders.

In various embodiments of the computer-implemented method, the mathematical model is parameterized using experimentally measured parameters or database parameters.

Preferably, the mathematical model for metabolic profiling is an algorithm for quantifying metabolic rates for at least one, preferably at least 5, more preferably at least 10 , even more preferably at least 15 and up to 25 central metabolic pathways, preferably selected from the following central metabolic pathways: (1) glycogen metabolism, (2) fructose metabolism, (3) galactose metabolism, (4) glycolysis, (5) gluconeogenesis, (6) oxidative pentose phosphate pathway, (7) non-oxidative pentose phosphate pathway, (8) fatty acid synthesis, (9) triglyceride synthesis, (10) synthesis and degradation of lipid droplets and synthesis of VLDL lipoprotein, (11) cholesterol synthesis, (12) tricarbonic acid (TCA) cycle, (13) respiratory chain and oxidative phosphorylation, (14) beta-oxidation of fatty acids, (15) urea cycle, (16) ethanol metabolism, (17) ketone body metabolism, (18) ammonia formation, (19) serine utilization, (20) alanine utilization, (21) branched chain amino acid metabolism, (22) branched-chain amino acid metabolism (BCAA), (23) glutamine metabolism, and (24) glutamate metabolism and (25) reactive oxygen species detoxification metabolism (ROS homeostasis).

In various embodiments, the algorithm used as the mathematical model for metabolic profiling is for quantifying the cellular energy metabolism by quantifying metabolic rates for respiratory chain and oxidative phosphorylation.

In various embodiments, the algorithm uses up to 618 protein/RNA expression levels selected from the those provided in Table 1 below. In various embodiments, the algorithm uses up to 113 protein/RNA expression levels of the respiratory chain & oxidative phosphorylation pathway selected from the those provided in Table 2 below. If the expression levels of these proteins/RNAs are not determined, they may be set to default or standard values. These values may be derived from experimental data, be obtained from public databases or determined from a reference cell or tissue.

The method may further comprise determining additional physicochemical input parameters and/or the expression level(s) of one or more further targets in the cell sample and providing the thus obtained data into the same mathematical model, in particular the mathematical model defined above.

In various embodiments, the one or more further targets are selected from targets in the oxidative phosphorylation pathway of a cell, more preferably one or more of:
(i) respiratory complex II and respiratory complex IV (as identified in Table 2);
(ii) respiratory complex II and respiratory complex V (as identified in Table 2); or
(iii) respiratory complex II and respiratory complex III (as identified in Table 2);
provided that not all of these targets are used in the method.

In various embodiments, the additional physicochemical input parameters are selected from glucose concentration, oxygen concentration, lactate concentration, ketone body concentration, and branched-chain amino acid (BCAA) concentration.

In various embodiments, the determination of the ANT expression level (and optionally further target expression level(s)) is carried out using methods and techniques known in the art. These include, without limitation, any one or more of mass spectrometry, Western blot, immunohistochemistry (IHC), ELISA, Immuno-PCR, Proximity Ligation Assay (PLA), aptamer assay, X-ray crystallography, NMR spectroscopy, cryo electron microscopy, protein microarray, gel electrophoresis, fluorescence in situ hybridization, qPCR, Northern blot, RNA microarray, RNA sequencing, single-cell RNA sequencing (scRNA-Seq), digital droplet PCR, branched DNA assays, nanostring, ribonuclease protection assay, poly(A) tail length assay, cap analysis of gene expression (CAGE), spatial genomics or spatial proteomics assay, flow cytometry, image cytometry and mass cytometry (CyTOF).

In various embodiments of the computer-implemented method, in step a) the expression levels of not all components involved in the metabolic oxidative phosphorylation pathway are determined and provided to the mathematical model.

In another aspect, the present invention relates to a computer program product configured to execute the computer-implemented method according to the invention on a computer. The computer program product is preferably configured to execute at least or only step c) of the inventive method. In other embodiments, it may be configured to execute steps b) and c). In embodiments where step a) draws the necessary information from a database, all steps may be executed by the computer program product.

### DETAILED DESCRIPTION OF THE INVENTION

Terms as set forth hereinafter are generally to be understood according to their common meaning as understood by those skilled in the art unless indicated otherwise.

The terms "include" and "comprising" do not exclude other elements and mean that there may be other components in addition to those mentioned. These terms are meant inclusively and therefore include "consisting of". "Consisting of" is meant conclusively and means that no further constituents may be present. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun, e.g., "a", "an" or "the", this includes a plural of that noun unless specifically stated otherwise.

The term "at least one" means numerically "one or more". In one embodiment, the term numerically means "one". In various other embodiments, "at least one" means one, two, three, four, five, six, seven, eight, nine or more, for example 10, 100 or 1000.

In a first aspect, the present invention relates to a computer-implemented method for determining the oxidative phosphorylation profile of a cell sample, the method comprising
a) determining the expression level(s) of adenine nucleotide translocator (ANT) and optionally one or more further targets involved in the oxidative metabolic phosphorylation pathway in the cell sample;
b) providing the expression level data obtained in step a) to a mathematical model for metabolic profiling; and
c) determining the oxidative phosphorylation profile of the cell sample (representative of its mitochondrial respiration profile) by calculation.

"Oxidative phosphorylation profile", as used herein, refers to the oxidative phosphorylation capability of the sample including but not necessarily limited to the oxidative phosphorylation rate. Said rate may, for example, be given as µmol ATP produced per g cells per time unit, for example per hours. The determination of the profile may include information on how production rate is influenced by various conditions.

"Adenine nucleotide translocator" or "ANT" is a protein of the inner mitochondrial membrane having four isoforms in humans, referred to as ANT1, ANT2, ANT3 and ANT4. It transports ADP from the cytosol into the mitochondria and exports ATP from the mitochondria to the cytosol. It is the only protein of complex VI of the oxidative phosphorylation pathway. If not indicated otherwise, all references to ANT made herein include all isoforms of ANT.

Surprisingly, the inventor of the present invention found that a significant determination of the ATP production rate in a cell sample of a subject is possible by determining the expression level of the protein ANT alone. However, in various embodiments, a combination of ANT and further targets is possible and can thus be also used. Specifically, it was found that using an established model for simulating the energy metabolism of a cell or tissue, the respiratory chain & oxidative phosphorylation pathway can be very reliably approximated by determining the expression level of ANT alone, also said pathway includes 112 different genes/proteins that are used for the simulation. The method described herein thus allows a much simpler process for determining the oxidative phosphorylation profile or ATP production rate of a sample cell of tissue, as it does not require determining the expression levels of all 112 proteins/genes or a substantial part thereof, but can be reliant on ANT expression levels alone essentially without compromising its accuracy and predictive potential.

In various embodiments of the computer-implemented method, the method may also include a step preceding step a) in which the cell sample is provided.

The sample may be a single cell sample. In other embodiments, it comprises a multitude of cells, for example in form of a cell suspension. The cell sample may alternatively also be an organoid. Also suitable are membrane-surrounded particles, including, but not limited to exosomes and extracellular vesicles. The cell sample may also be a tissue sample. This includes biopsy samples and spatial tissue sections. The cell sample may be obtained from an organism, typically a subject. Steps a) to c) of the inventive method are performed ex *vivo.*

In various embodiments, the cell sample can be a cell sample of living cells, dead cells and/or fixated cells, such as FFPE, frozen or freeze-dried cells.

In various embodiments, the oxidative phosphorylation profile is determined at single-cell, several cells (bulk), or spatial scale (spatial biology). Single cell determination may, for example, be carried out for immune cells, such as to determine an immune cell metabolic deficiency. Bulk scale determination is typically carried out on biopsies. Spatial scale is typically used in pathology. The determination of the oxidative phosphorylation profile at these levels typically requires that step a) is performed at the same level, e.g. if single cell analysis is desired, the expression level needs to be determined for a single cell.

It is preferred that the cell sample is a mammalian cell sample. Mammalian cell samples preferably include cell samples from human, mouse, rat, rabbit, pig or dog, without being limited thereto. In a preferred embodiment, the cell sample is a sample from human or mouse, in particular a human cell sample.

In various embodiments, the cell sample has been obtained from a subject, preferably a mammal, more preferably a human, mouse, rat, rabbit, pig, or dog, without being limited thereto, more preferably a human or mouse, in particular, the cell sample is obtained from a human subject.

In various embodiments of the computer-implemented method, the expression level is determined by determining the total mRNA level and/or protein level of ANT and all isoforms thereof in the sample. In various embodiments, the total mRNA level expressed from the ANT gene is determined. It has surprisingly been found that expression levels can be determined on mRNA level and that the results obtained correlated well with the protein levels. Alternatively or additionally, expression levels may be determined on protein level. Here, typically the total level of ANT in the cell sample including all isoforms is determined.

In various embodiments, the method further comprises determining the metabolic potential and/or the energy phenotype of the cell sample from the determined oxidative phosphorylation profile. The term "metabolic potential", as used in this context, means the estimated abundances of multiple metabolic functions in the cell sample and also covers the capability of a cell or a number of cells to support a shift from resting to activation and therefore combines the energy profiles at basal and maximum mitochondrial respiration. The term "energetic phenotype", as used herein, relates to define a cell's energy phenotype profile by determining mitochondrial respiration and glycolysis as well as energetic sources (e.g. carbohydrates, fatty acids, amino acids, or intracellular stores) under baseline (resting) and energetic stressed conditions (activated) to reveal key parameters of cell energy metabolism.

The method may further comprise the step of comparing the determined oxidative phosphorylation profile or metabolic potential of the cell sample to a reference profile or potential, originating, e.g., from reference cells or tissue. The reference profile may be the profile of a normal healthy cell or an abnormal, for example a diseased cell. If the sample is a tissue or biopsy, the reference may accordingly be a healthy or diseased tissue. The reference profile may be experimentally determined, for example in parallel, or may be taken from a database. The reference profile may also be artificially generated, for example by a multitude of experimental measurements that are normalized or averaged to yield the reference profile. Also possible is using a reference profile that is a desired profile.

In various embodiments, the determined oxidative phosphorylation profile of a diseased subject (patient, affected) can be compared to the oxidative phosphorylation profile of a non-diseased subject (control, normal).

In various embodiments, the difference between the sample profile and the reference profile is
(a) indicative for a disease or disorder that affects the oxidative phosphorylation profile of a cell;
(b) used to determine susceptibility to a specific treatment of a disease or disorder;
(c) used for risk stratification to develop a disease or disorder;
(d) used to monitor the progression or treatment of a disease or disorder;
(e) used to screen potential pharmaceutical actives for their pharmaceutical activity, safety, and/or metabolism;
(f) used to determine the age, nutritional status and/or overall health of a subject; and/or
(g) used to determine the inflammation status, infection status, hereditary disease status, epidemiologic status, environmental harm, or intoxication status of a subject.

Typically, the comparison allows to determine changes and aberrations in the oxidative phosphorylation pathway of the cell sample. Taken as such they may be indicative for a deviation from the normal state, but to allow any one of the conclusions listed under (a) to (g) above, additional parameters may need to be determined.

The disease or disorder may be selected from the group of neurodegenerative diseases or disorders, proliferative diseases or disorders, infectious diseases, oncologic diseases, mental disorders, cardiologic diseases and disorders, immunologic diseases and disorders, inflammation and metabolic diseases or disorders.

In various embodiments and without limitation, the disease or disorder may be selected from amyotrophic lateral sclerosis (ALS), Alzheimer's disease, Parkinson's disease, cancer, mitochondrial encephalomyopathy, medulloblastoma, cardiomyopathy, or obesity.

In various embodiments of the computer-implemented method, the mathematical model is parameterized using experimentally measured parameters, database parameters, or data from published literature. In various embodiments, using assumed parameters enables the simulation of oxidative phosphorylation potential under various assumed conditions (as described in the Examples).

Preferably, the mathematical model for metabolic profiling is an algorithm for quantifying metabolic rates for at least one, preferably at least 5, more preferably at least 10 , even more preferably at least 15 and up to 25 central metabolic pathways, preferably selected from the following central metabolic pathways: (1) glycogen metabolism, (2) fructose metabolism, (3) galactose metabolism, (4) glycolysis, (5) gluconeogenesis, (6) oxidative pentose phosphate pathway, (7) non-oxidative pentose phosphate pathway, (8) fatty acid synthesis, (9) triglyceride synthesis, (10) synthesis and degradation of lipid droplets and synthesis of VLDL lipoprotein, (11) cholesterol synthesis, (12) tricarbonic acid (TCA) cycle, (13) respiratory chain and oxidative phosphorylation, (14) beta-oxidation of fatty acids, (15) urea cycle, (16) ethanol metabolism, (17) ketone body metabolism, (18) ammonia formation, (19) serine utilization, (20) alanine utilization, (21) branched chain amino acid metabolism, (22) branched-chain amino acid metabolism (BCAA), (23) glutamine metabolism, and (24) glutamate metabolism and (25) reactive oxygen species detoxification metabolism (ROS homeostasis).

In various embodiments, the algorithm used as the mathematical model for metabolic profiling is for quantifying the cellular energy metabolism, for example by quantifying metabolic rates for respiratory chain and oxidative phosphorylation.

In various embodiments, the algorithm uses up to 618 protein/mRNA expression levels selected from the those provided in Table 1 below. These proteins/genes have been found to be involved and to a certain extent representative for the above-listed central metabolic pathways. As said list includes all four ANT isoforms, it is understood that the expression level(s) thereof can be determined in step a) of the inventive method and then entered to the mathematical model, i.e. the algorithm. For all other proteins/genes listed experimental values or, alternatively, database or assumed or default values may be used. As described above, it has been found that by only entering the ANT expression levels into the model, the respiratory chain and oxidative phosphorylation pathway may be highly accurately determined/simulated for the sample cell or tissue, i.e. thus obviating the need to determine all 112 protein/gene expression levels that are involved in the respiratory chain and oxidative phosphorylation pathway. Simulating or determining this part of the model is already valuable for a variety of different applications and uses, as further detailed herein below. However, if the complete model is to be used for determining the metabolic potential or energy phenotype of a cell or tissue, as defined above, additional protein/gene levels representative for the other 24 metabolic pathways may be determined, derived from a database or reference cell/tissue or may be set to default/unchanged relative to a reference. It is however understood that the property of ANT to allow simulating/determining the respiratory chain and oxidative phosphorylation pathway is unprecedented in that it cannot be expected that such representative single "markers" exist for all 24 remaining pathways. To provide an accurate complete model that considers all 25 relevant metabolic pathways, a multitude of additional gene/protein expression levels from the other 24 pathways may be determined. In various embodiments, ANT expression levels are determined as being representative for the respiratory chain and oxidative phosphorylation pathway and in addition up to 506 of the other gene/protein levels involved in different pathways are used, for example at least 50, at least 100, at least 150, at least 200, at least 250, at least 300, at least 350, at least 400 or at least 450 of these gene/protein expression levels. The "506 gene/protein levels involved in different pathways" are those listed in Table 1 below but not listed in Table 2 below.

It is understood that the algorithm may use not all of the indicated protein/mRNA expression levels, but only parts thereof. However, in various embodiments, the algorithm uses at least 100, preferably at least 150, at least 200, at least 250, at least 300, at least 350, at least 400, at least 450, at least 500, at least 550 or at least 600 of the indicated protein/mRNA expression levels. In various embodiments, it is however preferred that for the oxidative phosphorylation part, not all protein/mRNA expression levels of targets involved in this metabolic pathway are used, but that only ANT or ANT in combination with a limited number of other proteins/genes of the oxidative phosphorylation are used.

**Table 1**

| **Uniprot** | **Protein Name** | **Gene Name** |
|---|---|---|
| **Q9HCL2** | GPAT1_HUMAN | GPAM;GPAT1;KIAA1560 |
| **Q6NU12** | GPAT2_HUMAN | GPAT2 |
| **Q53EU6** | GPAT3_HUMAN | GPAT3;AGPAT9;MAG1 |
| **Q86UL3** | GPAT4_HUMAN | GPAT4;AGPAT6;TSARG7 |
| **Q14693** | LPIN1_HUMAN | LPIN1;KIAA0188 |
| **Q92539** | LPIN2_HUMAN | LPIN2;KIAA0249 |
| **Q9BQK8** | LPIN3_HUMAN | LPIN3;LIPN3L |
| **O14494** | PLPP1_HUMAN | PLPP1;LPP1;PPAP2A |
| **O43688** | PLPP2_HUMAN | PLPP2;LPP2;PPAP2C |
| **O14495** | PLPP3_HUMAN | PLPP3;LPP3;PPAP2B |
| **Q5VZY2** | PLPP4_HUMAN | PLPP4;DPPL2;PPAPDC1 ;PPAPDC1A |
| **Q8NEB5** | PLPP5_HUMAN | PLPP5;DPPL1 ;HTPAP;PPAPDC1B |
| **Q8TBJ4** | PLPR1_HUMAN | PLPPR1 ;LPPR1 ;PRG3 |
| **Q96GM1** | PLPR2_HUMAN | PLPPR2;LPPR2;PRG4 |
| **Q6T4P5** | PLPR3_HUMAN | PLPPR3;LPPR3;PHP2;PRG2 |
| **Q7Z2D5** | PLPR4_HUMAN | PLPPR4;LPPR4;KIAA0455;PHP1;PRG1 |
| **Q32ZL2** | PLPR5_HUMAN | PLPPR5;LPPR5;PAP2D;PRG5 |
| **Q99943** | PLCA_HUMAN | AGPAT1;G15 |
| **O15120** | PLCB_HUMAN | AGPAT2 |
| **Q9NRZ7** | PLCC_HUMAN | AGPAT3;LPAAT3 |
| **Q9NRZ5** | PLCD_HUMAN | AGPAT4 |
| **Q9NUQ2** | PLCE_HUMAN | AGPAT5 |
| **Q643R3** | LPCT4_HUMAN | LPCAT4;AGPAT7;AYTL3;LPEAT2 |
| **Q6UWP7** | LCLT1_HUMAN | LCLAT1 ;AGPAT8;ALCAT1 ;LYCAT |
| **Q8WTS1** | ABHD5_HUMAN | ABHD5;NCIE2 |
| **Q9NST1** | PLPL3_HUMAN | PNPLA3;ADPN;C22orf20 |
| **O75907** | DGAT1_HUMAN | DGAT1;AGRP1;DGAT |
| **Q96PD7** | DGAT2_HUMAN | DGAT2 |
| **Q6ZPD8** | DG2L6_HUMAN | DGAT2L6;DC3 |
| **Q86VF5** | MOGT3_HUMAN | MOGAT3;DC7;DGAT2L7 |
| **Q99685** | MGLL_HUMAN | MGLL |
| **Q9BV23** | ABHD6_HUMAN | ABHD6 |
| **Q8N2K0** | ABD12_HUMAN | ABHD12;C20orf22 |
| **Q7Z5M8** | AB12B_HUMAN | ABHD12B;C14orf29 |
| **Q99624** | S38A3_HUMAN | SLC38A3;G17;NAT1;SN1;SNAT3 |
| **Q99624** | S38A3_HUMAN | SLC38A3;G17;NAT1;SN1 ;SNAT3 |
| **O94925** | GLSK_HUMAN | GLS;GLS1;KIAA0838 |
| **Q9UI32** | GLSL_HUMAN | GLS2;GA |
| **P00367** | DHE3_HUMAN | GLUD1;GLUD |
| **P49448** | DHE4_HUMAN | GLUD2;GLUDP1 |
| **Q8N159** | NAGS_HUMAN | NAGS |
| **Q03154** | ACY1_HUMAN | ACY1 |
| **Q9H936** | GHC1_HUMAN | SLC25A22;GC1 |
| **Q9H1K4** | GHC2_HUMAN | SLC25A18;GC2 |
| **Q9NUB1** | ACS2L_HUMAN | ACSS1 ;ACAS2L; KIAA 1846 |
| **Q9NR19** | ACSA_HUMAN | ACSS2;ACAS2 |
| **Q9H6R3** | ACSS3_HUMAN | ACSS3 |
| **Q15181** | IPYR_HUMAN | PPA1;IOPPP;PP |
| **Q9H2U2** | IPYR2_HUMAN | PPA2 |
| **Q86TP1** | PRUN1_HUMAN | PRUNE1 |
| **Q9H008** | LHPP_HUMAN | LHPP |
| **P31327** | CPSM_HUMAN | CPS1 |
| **P00480** | OTC_HUMAN | OTC |
| **Q9Y619** | ORNT1_HUMAN | SLC25A15;ORNT1 |
| **Q9BXI2** | ORNT2_HUMAN | SLC25A2;ORNT2 |
| **P00966** | ASSY_HUMAN | ASS1;ASS |
| **P04424** | ARLY_HUMAN | ASL |
| **P05089** | ARGI1_HUMAN | ARG1 |
| **P17174** | AATC_HUMAN | GOT1 |
| **P00505** | AATM_HUMAN | GOT2;KYAT4 |
| **Q02978** | M2OM_HUMAN | SLC25A11 ;SLC20A4 |
| **O75746** | CMC1_HUMAN | SLC25A12;ARALAR1 |
| **Q9UJS0** | CMC2_HUMAN | SLC25A13;ARALAR2 |
| **P50416** | CPT1A_HUMAN | CPT1A;CPT1 |
| **Q92523** | CPT1B_HUMAN | CPT1B;KIAA1670 |
| **Q8TCG5** | CPT1C_HUMAN | CPT1C;CATL1 |
| **O43772** | MCAT_HUMAN | SLC25A20;CAC;CACT |
| **Q8N8R3** | MCATL_HUMAN | SLC25A29;C14orf69;ORNT3 |
| **P23786** | CPT2_HUMAN | CPT2;CPT1 |
| **P16219** | ACADS_HUMAN | ACADS |
| **P11310** | ACADM_HUMAN | ACADM |
| **P28330** | ACADL_HUMAN | ACADL |
| **P49748** | ACADV_HUMAN | ACADVL;VLCAD |
| **P30084** | ECHM_HUMAN | ECHS1 |
| **Q16836** | HCDH_HUMAN | HADH;HAD;HAD1;HADHSC;SCHAD |
| **P40939** | ECHA_HUMAN | HADHA;HADH |
| **Q99714** | HCD2_HUMAN | HSD17B10;ERAB;HADH2;MRPP2;SCHAD;SDR5C1;XH98G2 |
| **P09110** | THIK_HUMAN | ACAA1;ACAA;PTHIO |
| **P42765** | THIM_HUMAN | ACAA2 |
| **P55084** | ECHB_HUMAN | HADHB;MSTP029 |
| **P24752** | THIL_HUMAN | ACAT1 ;ACAT;MAT |
| **P08559** | ODPA_HUMAN | PDHA1;PHE1A |
| **P29803** | ODPAT_HUMAN | PDHA2;PDHAL |
| **P11177** | ODPB_HUMAN | PDHB;PHE1B |
| **P10515** | ODP2_HUMAN | DLAT;DLTA |
| **P09622** | DLDH_HUMAN | DLD;GCSL;LAD;PHE3 |
| **O75390** | CISY_HUMAN | CS |
| **P21399** | ACOC_HUMAN | ACO1;IREB1 |
| **Q99798** | ACON_HUMAN | ACO2 |
| **P50213** | IDH3A_HUMAN | IDH3A |
| **O43837** | IDH3B_HUMAN | IDH3B |
| **P51553** | IDH3G_HUMAN | IDH3G |
| **O75874** | IDHC_HUMAN | IDH1;PICD |
| **P48735** | IDHP_HUMAN | IDH2 |
| **Q02218** | ODO1_HUMAN | OGDH |
| **Q96HY7** | DHTK1_HUMAN | DHTKD1;KIAA1630 |
| **P36957** | ODO2_HUMAN | DLST;DLTS |
| **P09622** | DLDH_HUMAN | DLD;GCSL;LAD;PHE3 |
| **P53597** | SUCA_HUMAN | SUCLG1 |
| **P53597** | SUCA_HUMAN | SUCLG1 |
| **Q96I99** | SUCB2_HUMAN | SUCLG2 |
| **Q9P2R7** | SUCB1_HUMAN | SUCLA2 |
| **P31040** | SDHA_HUMAN | SDHA;SDH2;SDHF |
| **P21912** | SDHB_HUMAN | SDHB;SDH;SDH1 |
| **Q99643** | C560_HUMAN | SDHC;CYB560;SDH3 |
| **O14521** | DHSD_HUMAN | SDHD;SDH4 |
| **P07954** | FUMH_HUMAN | FH |
| **P40925** | MDHC_HUMAN | MDH1;MDHA |
| **Q5I0G3** | MDH1B_HUMAN | MDH1B |
| **P40926** | MDHM_HUMAN | MDH2 |
| **O14561** | ACPM_HUMAN | NDUFAB1 |
| **O15239** | NDUA1_HUMAN | NDUFA1 |
| **O43678** | NDUA2_HUMAN | NDUFA2 |
| **O95167** | NDUA3_HUMAN | NDUFA3 |
| **O00483** | NDUA4_HUMAN | NDUFA4 |
| **Q9NRX3** | NUA4L_HUMAN | NDUFA4L2 |
| **Q16718** | NDUA5_HUMAN | NDUFA5 |
| **P56556** | NDUA6_HUMAN | NDUFA6;LYRM6;NADHB14 |
| **095182** | NDUA7_HUMAN | NDUFA7 |
| **P51970** | NDUA8_HUMAN | NDUFA8 |
| **Q16795** | NDUA9_HUMAN | NDUFA9;NDUFS2L |
| **O95299** | NDUAA_HUMAN | NDUFA10 |
| **Q86Y39** | NDUAB_HUMAN | NDUFA11 |
| **Q9UI09** | NDUAC_HUMAN | NDUFA12;DAP13 |
| **Q8N183** | NDUF2_HUMAN | NDUFAF2;NDUFA12L |
| **Q9P0J0** | NDUAD_HUMAN | NDUFA13;GRIM19 |
| **Q9BU61** | NDUF3_HUMAN | NDUFAF3 |
| **Q9P032** | NDUF4_HUMAN | NDUFAF4;C6orf661 ;HRPAP20 |
| **Q5TEU4** | NDUF5_HUMAN | NDUFAF5;C20orf7 |
| **O75438** | NDUB1_HUMAN | NDUFB1 |
| **O95178** | NDUB2_HUMAN | NDUFB2 |
| **O43676** | NDUB3_HUMAN | NDUFB3 |
| **O95168** | NDUB4_HUMAN | NDUFB4 |
| **O43674** | NDUB5_HUMAN | NDUFB5 |
| **O95139** | NDUB6_HUMAN | NDUFB6 |
| **P17568** | NDUB7_HUMAN | NDUFB7 |
| **O95169** | NDUB8_HUMAN | NDUFB8 |
| **Q9Y6M9** | NDUB9_HUMAN | NDUFB9;LYRM3;UQOR22 |
| **O96000** | NDUBA_HUMAN | NDUFB10 |
| **Q9NX14** | NDUBB_HUMAN | NDUFB11 |
| **O43677** | NDUC1_HUMAN | NDUFC1 |
| **O95298** | NDUC2_HUMAN | NDUFC2 |
| **E9PQ53** | NDUCR_HUMAN | NDUFC2-KCTD14 |
| **P49821** | NDUV1_HUMAN | NDUFV1;UQOR1 |
| **P19404** | NDUV2_HUMAN | NDUFV2 |
| **P56181** | NDUV3_HUMAN | NDUFV3 |
| **P28331** | NDUS1_HUMAN | NDUFS1 |
| **O75306** | NDUS2_HUMAN | NDUFS2 |
| **O75489** | NDUS3_HUMAN | NDUFS3 |
| **O43181** | NDUS4_HUMAN | NDUFS4 |
| **O43920** | NDUS5_HUMAN | NDUFS5 |
| **O75380** | NDUS6_HUMAN | NDUFS6 |
| **075251** | NDUS7_HUMAN | NDUFS7 |
| **O00217** | NDUS8_HUMAN | NDUFS8 |
| **P03886** | NU1M_HUMAN | MT-ND1;MTND1;NADH1;ND1 |
| **P03891** | NU2M_HUMAN | MT-ND2;MTND2;NADH2;ND2 |
| **P03897** | NU3M_HUMAN | MT-ND3;MTND3;NADH3;ND3 |
| **P03905** | NU4M_HUMAN | MT-ND4;MTND4;NADH4;ND4 |
| **P03901** | NU4LM_HUMAN | MT-ND4L;MTND4L;NADH4L;ND4L |
| **P03915** | NU5M_HUMAN | MT-ND5;MTND5;NADH5;ND5 |
| **P03923** | NU6M_HUMAN | MT-ND6;MTND6;NADH6;ND6 |
| **P31930** | QCR1_HUMAN | UQCRC1 |
| **P22695** | QCR2_HUMAN | UQCRC2 |
| **P00156** | CYB_HUMAN | MT-CYB;COB;CYTB;MTCYB |
| **P08574** | CY1_HUMAN | CYC1 |
| **P47985** | UCRI_HUMAN | UQCRFS1 |
| **P07919** | QCR6_HUMAN | UQCRH |
| **P14927** | QCR7_HUMAN | UQCRB;UQBP |
| **O14949** | QCR8_HUMAN | UQCRQ |
| **Q9UDW1** | QCR9_HUMAN | UQCR10;UCRC |
| **O14957** | QCR10_HUMAN | UQCR11;UQCR |
| **P99999** | CYC_HUMAN | CYCS;CYC |
| **P25705** | ATPA_HUMAN | ATP5F1A;ATP5A;ATP5A1;ATP5AL2;ATPM |
| **P06576** | ATPB_HUMAN | ATP5F1B;ATP5B;ATPMB;ATPSB |
| **P36542** | ATPG_HUMAN | ATP5F1 C;ATP5C;ATP5C1 ;ATP5CL1 |
| **P30049** | ATPD_HUMAN | ATP5F1D;ATP5D |
| **P56381** | ATP5E_HUMAN | ATP5F1E;ATP5E |
| **Q5VTU8** | AT5EL_HUMAN | ATP5F1EP2;ATP5EP2 |
| **P00846** | ATP6_HUMAN | MT-ATP6;ATP6;ATPASE6;MTATP6 |
| **P24539** | AT5F1_HUMAN | ATP5PB;ATP5F1 |
| **P05496** | AT5G1_HUMAN | ATP5MC1;ATP5G1 |
| **Q06055** | AT5G2_HUMAN | ATP5MC2;ATP5G2 |
| **P48201** | AT5G3_HUMAN | ATP5MC3;ATP5G3 |
| **O75947** | ATP5H_HUMAN | ATP5PD;ATP5H |
| **P56385** | ATP5I_HUMAN | ATP5ME;ATP5I;ATP5K |
| **P18859** | ATP5J_HUMAN | ATP5PF;ATP5A;ATP5J;ATPM |
| **P56134** | ATPK_HUMAN | ATP5MF;ATP5J2;ATP5JL |
| **O75964** | ATP5L_HUMAN | ATP5MG;ATP5L |
| **Q7Z4Y8** | AT5L2_HUMAN | ATP5MGL;ATP5K2;ATP5L2 |
| **P03928** | ATP8_HUMAN | MT-ATP8;ATP8;ATPASE8;MTATP8 |
| **Q99766** | ATP5S_HUMAN | DMAC2L;ATP5S;ATPW |
| **Q9NW81** | DMAC2_HUMAN | DMAC2;ATP5SL |
| **P48047** | ATPO_HUMAN | ATP5PO;ATP50;ATPO |
| **P56378** | ATP68_HUMAN | ATP5MJ;ATP5MPL;C14orf2;MP68 |
| **Q00325** | MPCP_HUMAN | SLC25A3;PHC |
| **P00395** | COX1_HUMAN | MT-CO1;COI;COXI;MTCO1 |
| **P00403** | COX2_HUMAN | MT-CO2;COII;COX2;COXII;MTCO2 |
| **P00414** | COX3_HUMAN | MT-CO3;COIII;COXIII;MTCO3 |
| **P13073** | COX41_HUMAN | COX4I1;COX4 |
| **Q96KJ9** | COX42_HUMAN | COX4I2;COX4L2 |
| **P20674** | COX5A_HUMAN | COX5A |
| **P10606** | COX5B_HUMAN | COX5B |
| **P12074** | CX6A1_HUMAN | COX6A1 ;COX6AL |
| **Q02221** | CX6A2_HUMAN | COX6A2;COX6A;COX6AH |
| **P14854** | CX6B1_HUMAN | COX6B1 ;COX6B |
| **Q6YFQ2** | CX6B2_HUMAN | COX6B2 |
| **P09669** | COX6C_HUMAN | COX6C |
| **P24310** | CX7A1_HUMAN | COX7A1 ;COX7AH |
| **P14406** | CX7A2_HUMAN | COX7A2;COX7AL |
| **O60397** | COX7S_HUMAN | COX7A2P2;COX7A3;COX7AL2;COX7AP2 |
| **P24311** | COX7B_HUMAN | COX7B |
| **Q8TF08** | CX7B2_HUMAN | COX7B2 |
| **P15954** | COX7C_HUMAN | COX7C |
| **P10176** | COX8A_HUMAN | COX8A;COX8;COX8L |
| **Q7Z4L0** | COX8C_HUMAN | COX8C |
| **O14548** | COX7R_HUMAN | COX7A2L;COX7AR;COX7RP |
| **P53007** | TXTP_HUMAN | SLC25A1 ;SLC20A3 |
| **P53396** | ACLY_HUMAN | ACLY |
| **Q13085** | ACACA_HUMAN | ACACA;ACAC;ACC1 ;ACCA |
| **O00763** | ACACB_HUMAN | ACACB;ACC2;ACCB |
| **O95822** | DCMC_HUMAN | MLYCD |
| **O95822-2** | DCMC_HUMAN | MLYCD |
| **O95822** | DCMC_HUMAN | MLYCD |
| **O95822-2** | DCMC_HUMAN | MLYCD |
| **P49327** | FAS_HUMAN | FASN;FAS |
| **P33121** | ACSL1_HUMAN | ACSL1 ;FACL1 ;FACL2;LACS;LACS1 ;LACS2 |
| **O95573** | ACSL3_HUMAN | ACSL3;ACS3;FACL3;LACS3 |
| **O60488** | ACSL4_HUMAN | ACSL4;ACS4;FACL4;LACS4 |
| **Q9ULC5** | ACSL5_HUMAN | ACSL5;ACS5;FACL5;UNQ633/PRO1250 |
| **Q9UKU0** | ACSL6_HUMAN | ACSL6;ACS2;FACL6; KIAA0837;LACS5 |
| **Q96GR2** | ACBG1_HUMAN | ACSBG1;BGM;KIAA0631;LPD |
| **Q5FVE4** | ACBG2_HUMAN | ACSBG2;BGR;UNQ2443/PRO5005 |
| **O14975** | S27A2_HUMAN | SLC27A2;ACSVL1;FACVL1;FATP2;VLACS |
| **Q5K4L6** | S27A3_HUMAN | SLC27A3;ACSVL3;FATP3 |
| **Q6P1M0** | S27A4_HUMAN | SLC27A4;ACSVL4;FATP4 |
| **Q6PCB7** | S27A1_HUMAN | SLC27A1;ACSVL5;FATP1 |
| **O14975** | S27A2_HUMAN | SLC27A2;ACSVL1;FACVL1;FATP2;VLACS |
| **Q5K4L6** | S27A3_HUMAN | SLC27A3;ACSVL3;FATP3;PSEC0067;UNQ367/PRO703 |
| **Q6P1M0** | S27A4_HUMAN | SLC27A4;ACSVL4;FATP4 |
| **Q9Y2P5** | S27A5_HUMAN | SLC27A5;ACSB;ACSVL6;FACVL3;FATP5 |
| **Q9Y2P4** | S27A6_HUMAN | SLC27A6;ACSVL2;FACVL2;FATP1 |
| **Q01581** | HMCS1_HUMAN | HMGCS1;HMGCS |
| **P54868** | HMCS2_HUMAN | HMGCS2 |
| **Q8TB92** | HMGC2_HUMAN | HMGCLL1 |
| **P35914** | HMGCL_HUMAN | HMGCL |
| **Q9BUT1** | BDH2_HUMAN | BDH2;DHRS6;UNQ6308/PRO20933 |
| **Q02338** | BDH_HUMAN | BDH1;BDH |
| **P55809** | SCOT1_HUMAN | OXCT1 |
| **P15104** | GLNA_HUMAN | GLUL;GLNS |
| **P15104** | GLNA_HUMAN | GLUL;GLNS |
| **P11166** | GTR1_HUMAN | SLC2A1;GLUT1 |
| **P11168** | GTR2_HUMAN | SLC2A2;GLUT2 |
| **P11169** | GTR3_HUMAN | SLC2A3;GLUT3 |
| **P14672** | GTR4_HUMAN | SLC2A4;GLUT4 |
| **P19367** | HXK1_HUMAN | HK1 |
| **P52789** | HXK2_HUMAN | HK2 |
| **P52789** | HXK2_HUMAN | HK2 |
| **P52790** | HXK3_HUMAN | HK3 |
| **P35557** | HXK4_HUMAN | GCK |
| **Q14397** | GCKR_HUMAN | GCKR |
| **P30613** | KPYR_HUMAN | PKLR;PK1;PKL |
| **P14618** | KPYM_HUMAN | PKM;PKM2;OIP3;PK2;PK3 |
| **Q15181** | IPYR_HUMAN | PPA1;IOPPP;PP |
| **Q9H2U2** | IPYR2_HUMAN | PPA2;HSPC124 |
| **P06744** | G6PI_HUMAN | GPI |
| **P36871** | PGM1_HUMAN | PGM1 |
| **Q96G03** | PGM2_HUMAN | PGM2;MSTP006 |
| **Q16851** | UGPA_HUMAN | UGP2 |
| **P54840** | GYS2_HUMAN | GYS2 |
| **P13807** | GYS1_HUMAN | GYS1 |
| **P46976** | GLYG_HUMAN | GYG1;GYG |
| **O15488** | GLYG2_HUMAN | GYG2 |
| **Q04446** | GLGB_HUMAN | GBE1 |
| **P35573** | GDE_HUMAN | AGL;GDE |
| **P06737** | PYGL_HUMAN | PYGL |
| **P11217** | PYGM_HUMAN | PYGM |
| **P11216** | PYGB_HUMAN | PYGB |
| **P35575** | G6PC_HUMAN | G6PC;G6PT |
| **Q9NQR9** | G6PC2_HUMAN | G6PC2;IGRP |
| **Q9BUM1** | G6PC3_HUMAN | G6PC3 |
| **O43826** | G6PT1_HUMAN | SLC37A4 |
| **O00476** | NPT4_HUMAN | SLC17A3 |
| **Q8TED4** | SPX2_HUMAN | SLC37A2 |
| **Q8NCC5** | SPX3_HUMAN | SLC37A3 |
| **P60174** | TPIS_HUMAN | TPI1 |
| **P04075** | ALDOA_HUMAN | ALDOA |
| **P05062** | ALDOB_HUMAN | ALDOB |
| **P09972** | ALDOC_HUMAN | ALDOC |
| **P04406** | G3P_HUMAN | GAPDH |
| **O14556** | G3PT_HUMAN | GAPDHS |
| **P00558** | PGK1_HUMAN | PGK1 |
| **P07205** | PGK2_HUMAN | PGKB;PGK2 |
| **P18669** | PGAM1_HUMAN | PGAM1;PGAMA |
| **P15259** | PGAM2_HUMAN | PGAM2;PGAMM |
| **Q8N0Y7** | PGAM4_HUMAN | PGAM4;PGAM3 |
| **P06733** | ENOA_HUMAN | ENO1L1;MBPB1;MPB1;ENO1 |
| **P09104** | ENOG_HUMAN | ENO2 |
| **P13929** | ENOB_HUMAN | ENO3 |
| **P35558** | PCKGC_HUMAN | PCK1;PEPCK1 |
| **Q16822** | PCKGM_HUMAN | PCK2;PEPCK2 |
| **P15531** | NDKA_HUMAN | NME1 |
| **P22392** | NDKB_HUMAN | NME2 |
| **Q13232** | NDK3_HUMAN | NME3 |
| **O00746** | NDKM_HUMAN | NME4;NM23D |
| **P56597** | NDK5_HUMAN | NME5 |
| **O75414** | NDK6_HUMAN | NME6 |
| **Q9Y5B8** | NDK7_HUMAN | NME7 |
| **P00568** | KAD1_HUMAN | AK1 |
| **P54819** | KAD2_HUMAN | AK2;ADK2 |
| **Q9UIJ7** | KAD3_HUMAN | AK3;AK3L1;AK6;AKL3L |
| **P27144** | KAD4_HUMAN | AK4 |
| **Q9Y6K8** | KAD5_HUMAN | AK5 |
| **Q9Y3D8** | KAD6_HUMAN | AK6 |
| **Q96M32** | KAD7_HUMAN | AK7 |
| **Q96MA6** | KAD8_HUMAN | AK8 |
| **P16118** | F261_HUMAN | PFKFB1;F6PK;PFRX |
| **O60825** | F262_HUMAN | PFKFB2 |
| **Q16875** | F263_HUMAN | PFKFB3 |
| **Q16877** | F264_HUMAN | PFKFB4 |
| **O60825** | F262_HUMAN | PFKFB2 |
| **Q16875** | F263_HUMAN | PFKFB3 |
| **Q16877** | F264_HUMAN | PFKFB4 |
| **O00757** | F16P2_HUMAN | FBP2 |
| **P09467** | F16P1_HUMAN | FBP1;FBP |
| **P17858** | K6PL_HUMAN | PFKL |
| **Q01813** | K6PP_HUMAN | PFKP;PFKF |
| **P08237** | K6PF_HUMAN | PFKM;PFKX |
| **P00338** | LDHA_HUMAN | LDHA |
| **P07195** | LDHB_HUMAN | LDHB |
| **P07864** | LDHC_HUMAN | LDHC;LDH3;LDHX |
| **Q86WU2** | LDHD_HUMAN | LDHD |
| **P11498** | PYC_HUMAN | PC |
| **O75390** | CISY_HUMAN | CS |
| **P08559** | ODPA_HUMAN | PDHA1 |
| **P29803** | ODPAT_HUMAN | PDHA2;PDHAL |
| **P11177** | ODPB_HUMAN | PDHB;PHE1B |
| **P10515** | ODP2_HUMAN | DLAT;DLTA |
| **P09622** | DLDH_HUMAN | DLD;GCSL;LAD;PHE3 |
| **O00330** | ODPX_HUMAN | PDHX;PDX1 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **Q02978** | M2OM_HUMAN | SLC25A11 |
| **Q9UBX3** | DIC_HUMAN | SLC25A10;DIC |
| **P53007** | TXTP_HUMAN | SLC25A1;SLC20A3 |
| **P32189** | GLPK_HUMAN | GK |
| **Q14410** | GLPK2_HUMAN | GK2;GKP2;GKTA |
| **Q14409** | GLPK3_HUMAN | GK3P;GKTB |
| **Q6ZS86** | GLPK5_HUMAN | GK5 |
| **P43304** | GPDM_HUMAN | GPD2 |
| **P21695** | GPDA_HUMAN | GPD1 |
| **Q8N335** | GPD1L_HUMAN | GPD1L |
| **P57057** | G6PT2_HUMAN | SLC37A1;G3PP |
| **P11413** | G6PD_HUMAN | G6PD |
| **O95479** | G6PE_HUMAN | H6PD;GDH |
| **O95336** | 6PGL_HUMAN | PGLS |
| **P52209** | 6PGD_HUMAN | PGD;PGDH |
| **Q96AT9** | RPE_HUMAN | RPE |
| **P49247** | RPIA_HUMAN | RPIA |
| **P37837** | TALDO_HUMAN | TALDO1 |
| **P29401** | TKT_HUMAN | TKT |
| **P51854** | TKTL1_HUMAN | TKTL1 |
| **Q9H0I9** | TKTL2_HUMAN | TKTL2 |
| **P29401** | TKT_HUMAN | TKT |
| **P51854** | TKTL1_HUMAN | TKTL1 |
| **Q9H0I9** | TKTL2_HUMAN | TKTL2 |
| **P60891** | PRPS1_HUMAN | PRPS1 |
| **P11908** | PRPS2_HUMAN | PRPS2 |
| **P21108** | PRPS3_HUMAN | PRPS1L1 |
| **Q14558** | KPRA_HUMAN | PRPSAP1 |
| **O60256** | KPRB_HUMAN | PRPSAP2 |
| **P06213** | INSR_HUMAN | INSR |
| **P47871** | GLR_HUMAN | GCGR |
| **P01308** | INS_HUMAN | INS |
| **P01275** | GLUC_HUMAN | GCG |
| **P35568** | IRS1_HUMAN | IRS1 |
| **Q9Y4H2** | IRS2_HUMAN | IRS2 |
| **O14654** | IRS4_HUMAN | IRS4 |
| **P14735** | IDE_HUMAN | IDE |
| **Q9Y259** | CHKB_HUMAN | CHKB;CHETK;CHKL |
| **P35790** | CHKA_HUMAN | ;CHKA;CHK;CKI |
| **Q9Y6K0** | CEPT1_HUMAN | ;CEPT1;PRO1101 |
| **Q8WUD6** | CHPT1_HUMAN | ;CHPT1;CPT1;MSTP022 |
| **Q9UBM1** | PEMT_HUMAN | ;PEMT;PEMPT;PNMT |
| **Q8TCT1** | PHOP1_HUMAN | PHOSPHO1 |
| **P04054** | PA21B_HUMAN | PLA2G1 B;PLA2;PLA2A;PPLA2 |
| **P53816** | HRSL3_HUMAN | ;PLA2G16;HRASLS3;HREV107 |
| **P47712** | PA24A_HUMAN | PLA2G4A;CPLA2;PLA2G4 |
| **P14555** | PA2GA_HUMAN | ;PLA2G2A;PLA2B;PLA2L;RASF-A |
| **O60733** | PLPL9_HUMAN | ;PLA2G6;PLPLA9 |
| **Q9UP65** | PA24C_HUMAN | ;PLA2G4C |
| **Q9NZ20** | PA2G3_HUMAN | ;PLA2G3 |
| **P0C869** | PA24B_HUMAN | PLA2G4B |
| **Q86XP0** | PA24D_HUMAN | PLA2G4D |
| **P39877** | PA2G5_HUMAN | ;PLA2G5 |
| **Q9UNK4** | PA2GD_HUMAN | PLA2G2D;SPLASH |
| **O15496** | PA2GX_HUMAN | PLA2G10 |
| **Q9BZM1** | PG12A_HUMAN | ;PLA2G12A;PLA2G12;FKSG38;UNQ2519/PRO6012 |
| **Q3MJ16** | PA24E_HUMAN | ;PLA2G4E |
| **Q68DD2** | PA24F_HUMAN | ;PLA2G4F |
| **Q9NZK7** | PA2GE_HUMAN | ;PLA2G2E |
| **Q9BZM2** | PA2GF_HUMAN | PLA2G2F |
| **Q8NF37** | PCAT1_HUMAN | ;LPCAT1 ;AYTL2;PFAAP3 |
| **Q7L5N7** | PCAT2_HUMAN | LPCAT2;AGPAT11 ;AYTL1 |
| **Q643R3** | LPCT4_HUMAN | LPCAT4;AGPAT7;AYTL3;LPEAT2 |
| **Q6P1A2** | MBOA5_HUMAN | LPCAT3;MBOAT5;OACT5 |
| **Q9HBU6** | EKI1_HUMAN | ;ETNK1;EKI1 |
| **Q9NVF9** | EKI2_HUMAN | ;ETNK2;EKI2;HMFT1716 |
| **Q99447** | PCY2_HUMAN | ;PCYT2 |
| **Q9Y6K0** | CEPT1_HUMAN | CEPT1;PRO1101 |
| **Q9C0D9** | EPT1_HUMAN | ;EPT1;KIAA1724;SELI |
| **Q9UG56** | PISD_HUMAN | PISD |
| **Q92903** | CDS1_HUMAN | CDS1;CDS |
| **O95674** | CDS2_HUMAN | CDS2 |
| **O14735** | CDIPT_HUMAN | ;CDIPT;PIS;PIS1 |
| **P48651** | PTSS1_HUMAN | ;PTDSS1;KIAA0024;PSSA |
| **Q9BVG9** | PTSS2_HUMAN | ;PTDSS2;PSS2 |
| **P23526** | SAHH_HUMAN | AHCY;SAHH |
| **O43865** | SAHH2_HUMAN | AHCYL1;DCAL;XPVKONA |
| **Q96HN2** | SAHH3_HUMAN | ;AHCYL2;KIAA0828 |
| **Q99707** | METH_HUMAN | ;MTR |
| **Q93088** | BHMT1_HUMAN | ;BHMT |
| **Q00266** | METK1_HUMAN | ;MAT1A;AMS1;MATA1 |
| **P31153** | METK2_HUMAN | ;MAT2A;AMS2;MATA2 |
| **P42898** | MTHR_HUMAN | ;MTHFR |
| **P24752** | THIL_HUMAN | ACAT1 |
| **Q9BWD1** | THIC_HUMAN | ACAT2 |
| **Q01581** | HMCS1_HUMAN | HMGCS1 |
| **P54868** | HMCS2_HUMAN | HMGCS2 |
| **P04035** | HMDH_HUMAN | HMGCR |
| **Q03426** | KIME_HUMAN | MVK |
| **Q15126** | PMVK_HUMAN | PMVK |
| **P53602** | MVD1_HUMAN | MVD;MPD |
| **Q13907** | IDI1_HUMAN | IDI1 |
| **Q9BXS1** | IDI2_HUMAN | IDI2 |
| **P14324** | FPPS_HUMAN | FDPS;FPS;KIAA1293 |
| **O95749** | GGPPS_HUMAN | GGPS1 |
| **P37268** | FDFT_HUMAN | FDFT1 |
| **Q14534** | ERG1_HUMAN | SQLE;ERG1 |
| **P48449** | ERG7_HUMAN | LSS;OSC |
| **P07327** | ADH1A_HUMAN | ADH1A;ADH1 |
| **P00325** | ADH1B_HUMAN | ADH1B;ADH2 |
| **P00256** | ADH1G_HUMAN | ADH1C;ADH3 |
| **P08319** | ADH4_HUMAN | ADH4 |
| **P11766** | ADHX_HUMAN | ADH5;ADHX;FDH |
| **P28332** | ADH6_HUMAN | ADH6 |
| **P40394** | ADH7_HUMAN | ADH7 |
| **P05091** | ALDH2_HUMAN | ALDH2;ALDM |
| **P30837** | AL1B1_HUMAN | ALDH1B1;ALDH5;ALDHX |
| **P43353** | AL3B1_HUMAN | ALDH3B1;ALDH7 |
| **P48448** | AL3B2_HUMAN | ALDH3B2;ALDH8 |
| **P53985** | MOT1_HUMAN | SLC16A1 |
| **O60669** | MOT2_HUMAN | SLC16A7;MCT2 |
| **O95907** | MOT3_HUMAN | SLC16A8;MCT3 |
| **O15427** | MOT4_HUMAN | SLC16A3;MCT4 |
| **O15374** | MOT5_HUMAN | SLC16A4;MCT4;MCT5 |
| **O15375** | MOT6_HUMAN | SLC16A5;MCT5;MCT6 |
| **O15403** | MOT7_HUMAN | SLC16A6;MCT6;MCT7 |
| **Q13423** | NNTM_HUMAN | NNT |
| **P12235** | ADT1_HUMAN | SLC25A4;AAC1;ANT1 |
| **P05141** | ADT2_HUMAN | SLC25A5;ANT2 |
| **P12236** | ADT3_HUMAN | SLC25A6;ANT3 |
| **Q9H0C2** | ADT4_HUMAN | SLC25A31 ;AAC4;ANT4;SFEC |
| **P21695** | GPDA_HUMAN | GPD1 |
| **Q8N335** | GPD1L_HUMAN | GPD1L;KIAA0089 |
| **P43304** | GPDM_HUMAN | GPD2 |
| **P24298** | ALAT1_HUMAN | GPT;AAT1;GPT1 |
| **Q8TD30** | ALAT2_HUMAN | GPT2;AAT2;ALT2 |
| **P55157** | MTP_HUMAN | MTTP;MTP |
| **P38571** | LICH_HUMAN | LIPA |
| **P19835** | CEL_HUMAN | CEL;BAL |
| **Q6PIU2** | NCEH1_HUMAN | NCEH 1 ;AADACL 1 ; KIAA 1363 |
| **Q05469** | LIPS_HUMAN | LIPE |
| **Q96AD5** | PLPL2_HUMAN | PNPLA2;ATGL;FP17548 |
| **P11168** | GTR2_HUMAN | SLC2A2;GLUT2 |
| **P50053** | KHK_HUMAN | KHK |
| **P05062** | ALDOB_HUMAN | ALDOB;ALDB |
| **Q3LXA3** | TKFC_HUMAN | TKFC;DAK |
| **P11168** | GTR2_HUMAN | SLC2A2;GLUT2 |
| **P51570** | GALK1_HUMAN | GALK1;GALK |
| **P07902** | GALT_HUMAN | GALT |
| **Q14376** | GALE_HUMAN | GALE |
| **Q13336** | UT1_HUMAN | SLC14A1;HUT11;JK;RACH1;UT1;UTE |
| **Q15849** | UT2_HUMAN | SLC14A2;HUT2;UT2 |
| **P00367** | DHE3_HUMAN | GLUD1;GLUD |
| **P49448** | DHE4_HUMAN | GLUD2;GLUDP1 |
| **Q9UPY5** | XCT_HUMAN | SLC7A11 |
| **Q8TCU3** | S7A13_HUMAN | SLC7A13;AGT1;XAT2 |
| **P05165** | PCCA_HUMAN | PCCA |
| **P05166** | PCCB_HUMAN | PCCB |
| **Q96PE7** | MCEE_HUMAN | MCEE |
| **P22033** | MUTA_HUMAN | MMUT;MUT |
| **P45954** | ACDSB_HUMAN | ACADSB |
| **P35610** | SOAT1_HUMAN | SOAT1;ACACT;ACACT1;SOAT;STAT |
| **O75908** | SOAT2_HUMAN | SOAT2;ACACT2 |
| **Q15392** | DHC24_HUMAN | DHCR24;KIAA0018 |
| **Q9UBM7** | DHCR7_HUMAN | DHCR7;D7SR |
| **O75845** | SC5D_HUMAN | SC5D;SC5DL |
| **Q15125** | EBP_HUMAN | EBP |
| **P56937** | DHB7_HUMAN | HSD17B7;SDR37C1;UNQ2563/PRO6243 |
| **Q15738** | NSDHL_HUMAN | NSDHL;H105E3 |
| **Q15800** | MSMO1_HUMAN | MSMO1 ;DESP4;ERG25;SC4MOL |
| **O76062** | ERG24_HUMAN | TM7SF2;ANG1 |
| **Q16850** | CP51A_HUMAN | CYP51A1;CYP51 |
| **Q9NUB1** | ACS2L_HUMAN | ACSS1;ACAS2L;KIAA1846 |
| **Q9NR19** | ACSA_HUMAN | ACSS2;ACAS2 |
| **Q9H6R3** | ACSS3_HUMAN | ACSS3 |
| **P25874** | UCP1_HUMAN | UCP1;SLC25A7;UCP |
| **P55851** | UCP2_HUMAN | UCP2;SLC25A8 |
| **P55916** | UCP3_HUMAN | UCP3;SLC25A9 |
| **O95847** | UCP4_HUMAN | SLC25A27;UCP4 |
| **O95258** | UCP5_HUMAN | SLC25A14;BMCP1;UCP5 |
| **P15121** | ALDR_HUMAN | AKR1B1;ALDR1 |
| **Q00796** | DHSO_HUMAN | SORD |
| **P05091** | ALDH2_HUMAN | ALDH2;ALDM |
| **P30837** | AL1B1_HUMAN | ALDH1B1;ALDH5;ALDHX |
| **P43353** | AL3B1_HUMAN | ALDH3B1;ALDH7 |
| **P48448** | AL3B2_HUMAN | ALDH3B2;ALDH8 |
| **Q81VS8** | GLCTK_HUMAN | GLYCTK;HBEBP4;LP5910 |
| **P15121** | ALDR_HUMAN | AKR1B1;ALDR1 |
| **Q9Y2S2** | CRYL1_HUMAN | CRYL1;CRY |
| **Q9Y2S2** | CRYL1_HUMAN | CRYL1;CRY |
| **O75191** | XYLB_HUMAN | XYLB |
| **P29218** | IMPA1_HUMAN | IMPA1;IMPA |
| **P54687** | BCAT1_HUMAN | BCAT1;BCT1;ECA39 |
| **O15382** | BCAT2_HUMAN | BCAT2;BCATM;BCT2;ECA40 |
| **P12694** | ODBA_HUMAN | BCKDHA |
| **P21953** | ODBB_HUMAN | BCKDHB |
| **P30084** | ECHM_HUMAN | ECHS1 |
| **Q6NVY1** | HIBCH_HUMAN | HIBCH |
| **P31937** | 3HIDH_HUMAN | HIBADH |
| **Q02252** | MMSA_HUMAN | ALDH6A1;MMSDH |
| **Q99714** | HCD2_HUMAN | HSD17B10;ERAB;HADH2;MRPP2;SCHAD;SDR5C1;XH98G2 |
| **P42765** | THIM_HUMAN | ACAA2 |
| **P26440** | IVD_HUMAN | IVD |
| **Q96RQ3** | MCCA_HUMAN | MCCC1;MCCA |
| **Q9HCC0** | MCCB_HUMAN | MCCC2;MCCB |
| **Q13825** | AUHM_HUMAN | AUH |
| **P35914** | HMGCL_HUMAN | HMGCL |
| **P05165** | PCCA_HUMAN | PCCA |
| **P05166** | PCCB_HUMAN | PCCB |
| **P22033** | MUTA_HUMAN | MMUT;MUT |
| **P04114** | APOB_HUMAN | APOB |
| **O60664** | PLIN3_HUMAN | PLIN3;M6PRBP1;TIP47; |
| **Q99541** | PLIN2_HUMAN | PLIN2 |
| **O60240** | PLIN1_HUMAN | PLIN1 |
| **Q8WTS1** | ABHD5_HUMAN | ABHD5 |
| **Q96AD5** | PLPL2_HUMAN | PNPLA2;ATGL;FP17548 |
| **Q96AQ7** | CIDEC_HUMAN | CIDEC |
| **Q05469** | LIPS_HUMAN | LIPE |
| **P55157** | MTP_HUMAN | MTTP;MTP |
| **P07738** | PMGE_HUMAN | BPGM |
| **P07738** | PMGE_HUMAN | BPGM |
| **P14550** | AK1A1_HUMAN | AKR1A1;ALDR1;ALR |
| **P00390** | GSHR_HUMAN | GSR;GLUR;GRD1 |
| **P07203** | GPX1_HUMAN | GPX1 |
| **P18283** | GPX2_HUMAN | GPX2 |
| **P22352** | GPX3_HUMAN | GPX3;GPXP |
| **P36969** | GPX4_HUMAN | GPX4 |
| **O75715** | GPX5_HUMAN | GPX5 |
| **P59796** | GPX6_HUMAN | GPX6 |
| **Q96SL4** | GPX7_HUMAN | GPX7 |
| **Q8TED1** | GPX8_HUMAN | GPX8 |
| **Q86VQ6** | TRXR3_HUMAN | TXNRD3;TGR;TRXR3 |
| **Q9NNW7** | TRXR2_HUMAN | TXNRD2;KIAA1652;TRXR2 |
| **Q16881** | TRXR1_HUMAN | TXNRD1;GRIM12;KDRF |
| **Q06830** | PRDX1_HUMAN | PRDX1 ;PAGA;PAGB;TDPX2 |
| **P32119** | PRDX2_HUMAN | PRDX2;NKEFB;TDPX1 |
| **P30048** | PRDX3_HUMAN | PRDX3;AOP1 |
| **Q13162** | PRDX4_HUMAN | PRDX4 |
| **P30044** | PRDX5_HUMAN | PRDX5;ACR1;SBBI10 |
| **P30041** | PRDX6_HUMAN | PRDX6;AOP2;KIAA0106 |

The algorithm using the expression levels of these proteins/genes has been described before. This algorithm is disclosed in the following 3 references, which are incorporated herein by reference in their entirety:
(1) Berndt et al., HEPATOKIN1 is a biochemistry-based model of liver metabolism for applications in medicine and pharmacology. Nat Commun 9, 2386 (2018). (https://doi.org/10.1038/s41467-018-04720-9)
(2) Berndt et al., CARDIOKIN1: Computational Assessment of Myocardial Metabolic Capability in Healthy Controls and Patients With Valve Diseases. Circulation 2021, 144, 1926-1939. (https://doi.org/10.1161/CIRCULATIONAHA.121.055646)
(3) Berndt et al., Physiology-Based Kinetic Modeling of Neuronal Energy Metabolism Unravels the Molecular Basis of NAD(P)H Fluorescence Transients. Journal of Cerebral Blood Flow & Metabolism. 2015;35(9):1494-1506. (doi:10.1038/jcbfm.2015.7).

The algorithm that can be used in the described methods can be freely downloaded as an executable SBML file at https://static-content.springer.com/esm/art%3A10.1038%2Fs41416-019-0659-3/MediaObjects/41416_2019_659_MOESM2_ESM.xml.

The algorithm may be adapted for different tissues and cells, as disclosed in the references above. However, the part of the algorithm relating to the oxidative phosphorylation part is essentially independent from tissue and/or cell type.

In various embodiments, the algorithm uses up to 112 protein/RNA expression levels of the respiratory chain & oxidative phosphorylation pathway selected from the those provided in Table 2. Again, as said Table includes all four ANT isoforms, it is understood that the expression level(s) thereof are to be determined in step a) of the inventive method and then entered to the mathematical model, i.e. the algorithm. For all other proteins/genes listed experimental values or, alternatively, database or assumed or default values may be used.

**Table 2**

| **Uniprot** | **Protein Name** | **Gene Name** | **Complex** |
|---|---|---|---|
| **O14561** | ACPM HUMAN | NDUFAB1 | I |
| **O15239** | NDUA1 HUMAN | NDUFA1 | I |
| **O43678** | NDUA2 HUMAN | NDUFA2 | I |
| **095167** | NDUA3 HUMAN | NDUFA3 | I |
| **O00483** | NDUA4 HUMAN | NDUFA4 | I |
| **Q9NRX3** | NUA4L HUMAN | NDUFA4L2 | I |
| **Q16718** | NDUA5 HUMAN | NDUFA5 | I |
| **P56556** | NDUA6 HUMAN | NDUFA6;LYRM6;NADHB14 | I |
| **O95182** | NDUA7 HUMAN | NDUFA7 | I |
| **P51970** | NDUA8 HUMAN | NDUFA8 | I |
| **Q16795** | NDUA9 HUMAN | NDUFA9;NDUFS2L | I |
| **O95299** | NDUAA HUMAN | NDUFA10 | I |
| **Q86Y39** | NDUAB_HUMAN | NDUFA11 | I |
| **Q9UI09** | NDUAC HUMAN | NDUFA12;DAP13 | I |
| **Q8N183** | NDUF2 HUMAN | NDUFAF2;NDUFA12L | I |
| **Q9P0J0** | NDUAD HUMAN | NDUFA13;GRIM19 | I |
| **Q9BU61** | NDUF3 HUMAN | NDUFAF3 | I |
| **Q9P032** | NDUF4 HUMAN | NDUFAF4;C6orf661 ;HRPAP20 | I |
| **Q5TEU4** | NDUF5 HUMAN | NDUFAF5;C20orf7 | I |
| **O75438** | NDUB1 HUMAN | NDUFB1 | I |
| **O95178** | NDUB2 HUMAN | NDUFB2 | I |
| **O43676** | NDUB3 HUMAN | NDUFB3 | I |
| **O95168** | NDUB4 HUMAN | NDUFB4 | I |
| **O43674** | NDUB5 HUMAN | NDUFB5 | I |
| **095139** | NDUB6 HUMAN | NDUFB6 | I |
| **P17568** | NDUB7 HUMAN | NDUFB7 | I |
| **095169** | NDUB8 HUMAN | NDUFB8 | I |
| **Q9Y6M9** | NDUB9 HUMAN | NDUFB9;LYRM3;UQOR22 | I |
| **O96000** | NDUBA HUMAN | NDUFB10 | I |
| **Q9NX14** | NDUBB HUMAN | NDUFB11 | I |
| **O43677** | NDUC1 HUMAN | NDUFC1 | I |
| **O95298** | NDUC2 HUMAN | NDUFC2 | I |
| **E9PQ53** | NDUCR HUMAN | NDUFC2-KCTD14 | I |
| **P49821** | NDUV1 HUMAN | NDUFV1;UQOR1 | I |
| **P19404** | NDUV2 HUMAN | NDUFV2 | I |
| **P56181** | NDUV3 HUMAN | NDUFV3 | I |
| **P28331** | NDUS1 HUMAN | NDUFS1 | I |
| **O75306** | NDUS2 HUMAN | NDUFS2 | I |
| **075489** | NDUS3 HUMAN | NDUFS3 | I |
| **043181** | NDUS4 HUMAN | NDUFS4 | I |
| **O43920** | NDUS5 HUMAN | NDUFS5 | I |
| **O75380** | NDUS6 HUMAN | NDUFS6 | I |
| **O75251** | NDUS7 HUMAN | NDUFS7 | I |
| **O00217** | NDUS8 HUMAN | NDUFS8 | I |
| **P03886** | NU1M HUMAN | MT-ND1;MTND1;NADH1;ND1 | I |
| **P03891** | NU2M HUMAN | MT-ND2;MTND2;NADH2;ND2 | I |
| **P03897** | NU3M HUMAN | MT-ND3;MTND3;NADH3;ND3 | I |
| **P03905** | NU4M HUMAN | MT-ND4;MTND4;NADH4;ND4 | I |
| **P03901** | NU4LM HUMAN | MT-ND4L;MTND4L;NADH4L;ND4L | I |
| **P03915** | NU5M HUMAN | MT-ND5;MTND5;NADH5;ND5 | I |
| **P03923** | NU6M HUMAN | MT-ND6;MTND6;NADH6;ND6 | I |
| **P31040** | SDHA HUMAN | SDHA;SDH2;SDHF | II |
| **P21912** | SDHB HUMAN | SDHB;SDH;SDH1 | II |
| **Q99643** | C560 HUMAN | SDHC;CYB560;SDH3 | II |
| **014521** | DHSD HUMAN | SDHD;SDH4 | II |
| **P31930** | QCR1 HUMAN | UQCRC1 | III |
| **P22695** | QCR2 HUMAN | UQCRC2 | III |
| **P00156** | CYB_HUMAN | MT-CYB;COB;CYTB;MTCYB | III |
| **P08574** | CY1_HUMAN | CYC1 | III |
| **P47985** | UCRI HUMAN | UQCRFS1 | III |
| **P07919** | QCR6 HUMAN | UQCRH | III |
| **P14927** | QCR7 HUMAN | UQCRB;UQBP | III |
| **O14949** | QCR8 HUMAN | UQCRQ | III |
| **Q9UDW1** | QCR9 HUMAN | UQCR10;UCRC | III |
| **O14957** | QCR10 HUMAN | UQCR11;UQCR | III |
| **P25705** | ATPA HUMAN | ATP5F1A;ATP5A;ATP5A1;ATP5AL2;ATPM | V |
| **P06576** | ATPB_HUMAN | ATP5F1B;ATP5B;ATPMB;ATPSB | V |
| **P36542** | ATPG HUMAN | ATP5F1C;ATP5C;ATP5C1 ;ATP5CL1 | V |
| **P30049** | ATPD HUMAN | ATP5F1D;ATP5D | V |
| **P56381** | ATP5E HUMAN | ATP5F1E;ATP5E | V |
| **Q5VTU8** | AT5EL HUMAN | ATP5F1EP2;ATP5EP2 | V |
| **P00846** | ATP6 HUMAN | MT-ATP6;ATP6;ATPASE6;MTATP6 | V |
| **P24539** | AT5F1 HUMAN | ATP5PB;ATP5F1 | V |
| **P05496** | AT5G1 HUMAN | ATP5MC1;ATP5G1 | V |
| **Q06055** | AT5G2 HUMAN | ATP5MC2;ATP5G2 | V |
| **P48201** | AT5G3 HUMAN | ATP5MC3;ATP5G3 | V |
| **O75947** | ATP5H HUMAN | ATP5PD;ATP5H | V |
| **P56385** | ATP5I HUMAN | ATP5ME;ATP5I;ATP5K | V |
| **P18859** | ATP5J HUMAN | ATP5PF;ATP5A;ATP5J;ATPM | V |
| **P56134** | ATPK HUMAN | ATP5MF;ATP5J2;ATP5JL | V |
| **O75964** | ATP5L HUMAN | ATP5MG;ATP5L | V |
| **Q7Z4Y8** | AT5L2 HUMAN | ATP5MGL;ATP5K2;ATP5L2 | V |
| **P03928** | ATP8 HUMAN | MT-ATP8;ATP8;ATPASE8;MTATP8 | V |
| **Q99766** | ATP5S HUMAN | DMAC2L;ATP5S;ATPW | V |
| **Q9NW81** | DMAC2 HUMAN | DMAC2;ATP5SL | V |
| **P48047** | ATPO_HUMAN | ATP5PO;ATP5O;ATPO | V |
| **P56378** | ATP68 HUMAN | ATP5MJ;ATP5MPL;C14orf2;MP68 | V |
| **P00395** | COX1 HUMAN | MT-CO1;COI;COXI;MTCO1 | IV |
| **P00403** | COX2 HUMAN | MT-CO2;COII;COX2;COXII;MTCO2 | IV |
| **P00414** | COX3 HUMAN | MT-CO3;COIII;COXIII;MTCO3 | IV |
| **P13073** | COX41 HUMAN | COX4I1;COX4 | IV |
| **Q96KJ9** | COX42 HUMAN | COX4I2;COX4L2 | IV |
| **P20674** | COX5A HUMAN | COX5A | IV |
| **P10606** | COX5B HUMAN | COX5B | IV |
| **P12074** | CX6A1 HUMAN | COX6A1 ;COX6AL | IV |
| **Q02221** | CX6A2 HUMAN | COX6A2;COX6A;COX6AH | IV |
| **P14854** | CX6B1 HUMAN | COX6B1;COX6B | IV |
| **Q6YFQ2** | CX6B2 HUMAN | COX6B2 | IV |
| **P09669** | COX6C HUMAN | COX6C | IV |
| **P24310** | CX7A1 HUMAN | COX7A1 ;COX7AH | IV |
| **P14406** | CX7A2 HUMAN | COX7A2;COX7AL | IV |
| **O60397** | COX7S HUMAN | COX7 A2P2;COX7 A3;COX7 AL2;COX7 AP2 | IV |
| **P24311** | COX7B HUMAN | COX7B | IV |
| **Q8TF08** | CX7B2 HUMAN | COX7B2 | IV |
| **P15954** | COX7C HUMAN | COX7C | IV |
| **P10176** | COX8A HUMAN | COX8A;COX8;COX8L | IV |
| **Q7Z4L0** | COX8C HUMAN | COX8C | IV |
| **O14548** | COX7R HUMAN | COX7A2L;COX7AR;COX7RP | IV |
| **P12235** | ADT1 HUMAN | SLC25A4;AAC1;ANT1 | VI |
| **P05141** | ADT2 HUMAN | SLC25A5;ANT2 | VI |
| **P12236** | ADT3 HUMAN | SLC25A6;ANT3 | VI |
| **Q9H0C2** | ADT4 HUMAN | SLC25A31 ;AAC4;ANT4;SFEC | VI |

The method may further comprise determining additional physicochemical input parameters, individual parameters, and/or the expression level(s) of one or more further targets in the cell sample and providing the thus obtained data to the mathematical model.

The oxidative phosphorylation (OXPHOS) is the central biological process responsible for energy production (ATP generation) and includes complexes I-IV that produce energy via the respiratory chain, complex V that converted the energy into chemical energy through chemiosmotic coupling and the transport protein ANT (complex VI).

In various embodiments, the expression level(s) of one or more target proteins/mRNAs other than ANT are determined and also provided/applied to the mathematical model. However, as ANT has been found to be representative for the oxidative phosphorylation, determination of these additional targets is typically not necessary to determine the oxidative phosphorylation profile of the cell sample. If such additional targets are used, their number is preferably limited to not more than 30, more preferably not more than 20, even more preferably not more than 10, for example 9, 8, 7, 6, 5, 4, 3, 2, or 1 additional target(s). In various preferred embodiments, the expression levels of not all of complexes I to VI are used in the inventive methods. In various preferred embodiments, the expression levels of only ANT are determined and provided to the mathematical model. This is consistent with the gist of the present invention, namely that ANT alone is sufficient to simulate/determine the oxidative phosphorylation profile of a cell or tissue.

In various embodiments, these additional targets are selected from proteins/genes in respiratory complex I, respiratory complex II, respiratory complex III, respiratory complex IV and/or respiratory complex V (as indicated in Table 2 above).

In various embodiments, one further target is selected from targets in the oxidative phosphorylation pathway of a cell, preferably from:
(i) respiratory complex I;
(ii) respiratory complex II;
(iii) respiratory complex III;
(iv) respiratory complex IV; or
(v) respiratory complex V.

In various embodiments, one or more further targets are selected from targets in the oxidative phosphorylation pathway of a cell, preferably from:
(i) respiratory complex I and respiratory complex II;
(ii) respiratory complex I and respiratory complex III;
(iii) respiratory complex I and respiratory complex IV;
(iv) respiratory complex I and respiratory complex V;
(v) respiratory complex II and respiratory complex III;
(vi) respiratory complex II and respiratory complex IV;
(vii) respiratory complex II and respiratory complex V;
(viii) respiratory complex III and respiratory complex IV;
(ix) respiratory complex III and respiratory complex V; or
(x) respiratory complex IV and respiratory complex V;
(xi) respiratory complex I, respiratory complex II and respiratory complex III;
(xii) respiratory complex I, respiratory complex II and respiratory complex IV;
(xiii) respiratory complex I, respiratory complex II and respiratory complex V;
(xiv) respiratory complex I, respiratory complex III and respiratory complex IV;
(xv) respiratory complex I, respiratory complex III and respiratory complex V;
(xvi) respiratory complex I, respiratory complex IV and respiratory complex V;
(xvii) respiratory complex II, respiratory complex III and respiratory complex IV;
(xviii) respiratory complex II, respiratory complex III and respiratory complex V;
(xix) respiratory complex II, respiratory complex IV and respiratory complex V;
(xx) respiratory complex III, respiratory complex IV and respiratory complex V;
(xxi) respiratory complex I, respiratory complex II, respiratory complex III and respiratory complex IV;
(xxii) respiratory complex I, respiratory complex II, respiratory complex III and respiratory complex V;
(xxiii) respiratory complex I, respiratory complex II, respiratory complex IV and respiratory complex V;
(xxiv) respiratory complex I, respiratory complex III, respiratory complex IV and respiratory complex V;
(xxv) respiratory complex II, respiratory complex III, respiratory complex IV and respiratory complex V;
(xxvi) respiratory complex I, respiratory complex II, respiratory complex III, respiratory complex IV and respiratory complex V.

Preferably, the one or more further targets are selected from targets in the oxidative phosphorylation pathway of a cell, more preferably one or more of:
(i) respiratory complex II and respiratory complex IV;
(ii) respiratory complex II and respiratory complex V; or
(iii) respiratory complex II and respiratory complex III;
provided that not all of these targets are used in the method.

In specific embodiments, the targets are ANT and one or more targets, preferably 1, 2, 3, 4, 5 or 6 targets, more preferably 1, 2 or 3 targets, from respiratory complex I.

In various other embodiments, the targets are ANT and one or more targets, preferably 1, 2, 3, 4, 5 or 6 targets, more preferably 1, 2 or 3 targets, from respiratory complex V.

In various embodiments, the targets are ANT and one or more targets, preferably 1, 2, 3, 4, 5 or 6 targets, more preferably 1, 2 or 3 targets, from respiratory complex I and one or more targets, preferably 1, 2, 3, 4, 5 or 6 targets, more preferably 1, 2 or 3 targets, from respiratory complex V.

In various other embodiments, the targets are ANT and one or more targets, preferably 1, 2, 3, 4, 5 or 6 targets, more preferably 1, 2 or 3 targets, from respiratory complex II, and one or more targets, preferably 1, 2, 3, 4, 5 or 6 targets, more preferably 1, 2 or 3 targets, from respiratory complex III.

In various other embodiments, the targets are ANT and one or more targets, preferably 1, 2, 3, 4, 5 or 6 targets, from respiratory complex II and one or more targets, preferably 1, 2, 3, 4, 5 or 6 targets, from respiratory complex IV.

In various other embodiments, the targets are ANT and one or more targets, preferably 1, 2, 3, 4, 5 or 6 targets, more preferably 1, 2 or 3 targets, from respiratory complex III and one or more targets, preferably 1, 2, 3, 4, 5 or 6 targets, more preferably 1, 2 or 3 targets, from respiratory complex IV.

In various embodiments, the additional physicochemical input parameters include (but are not limited to): glucose concentration, oxygen concentration, lactate concentration, ketone body concentration, and branched-chain amino acid (BCAA) concentration.

Key output parameters include (but are not limited to): physicochemical parameters selected from oxygen consumption rate; acidification rate; ATP production rate; mitochondrial membrane potential; reactive oxygen species (ROS) levels; glucose uptake rate; lactate production rates; exchange fluxes for fatty acids, glycerol, BCAAs, ketone bodies, and other amino acids; redox states (NAD/NADH, NADP/NADPH, FAD/FADH2) in the cytosol, mitochondria, or even resolved enzymatically; glycogen content; triacylglycerol (TAG) content; mitochondrial pH; and ion concentrations (sodium, potassium, calcium) in the cytosol and mitochondria.

In various embodiments, further individual input parameters can be used for analysis. These individual parameters include, without being limited thereto, patent age, smoking behavior, systolic and/or diastolic blood pressure, HDL cholesterol level, blood glucose concentration, triglyceride concentration, subject sex, and medication.

In various embodiments, the determination of the ANT and optionally further target expression level is carried out using any one or more of mass spectrometry, Western blot, immunohistochemistry (IHC), ELISA (enzyme-linked immuno sorbent assay), Immuno-PCR, Proximity Ligation Assay (PLA), immunohistochemical staining, in situ hybridization (ISH), loop-mediated isothermal amplification (LAMP), immunoprecipitation, radio immuno assay (RIA), fluorescence-activated cell sorting (FACS), visual inspection aptamer assay, X-ray crystallography, NMR spectroscopy, cryo electron microscopy, protein microarray, gel electrophoresis, Fluorescence In Situ Hybridization, quantitative Polymerase Chain Reaction (qPCR) Reverse Transkription Polymerase Chain Reaction (RT-PCR), quantitative Real-Time PCR (qRT-PCR), Northern blot, (RNA) microarray, RNA-sequencing (RNA-Seq), Single-Cell RNA Sequencing (scRNA-Seq), digital droplet PCR, branched DNA assays, Nanostring, ribonuclease protection assay, poly(A) tail length assay, single cell proteomics, cap analysis of gene expression (CAGE), spatial genomics or spatial proteomics assay, flow cytometry, image cytometry and mass cytometry (CyTOF).

Preferably, the determination of the ANT and optionally further target expression level is carried out using any one or more of mass spectrometry, Western blot, IHC, ELISA, Immuno-PCR, Proximity Ligation Assay (PLA), aptamer assay, X-ray crystallography, NMR spectroscopy, cryo electron microscopy, protein microarray, gel electrophoresis, Fluorescence In Situ Hybridization, qPCR, Northern blot, RNA microarray, RNA-sequencing (RNA-Seq), Single-Cell RNA Sequencing (scRNA-Seq), digital droplet PCR, branched DNA assays, Nanostring, ribonuclease protection assay, poly(A) tail length assay, cap analysis of gene expression (CAGE), spatial genomics or spatial proteomics assay, flow cytometry, image cytometry and mass cytometry (CyTOF).

The properties, RNA sequences and amino acid sequences of ANT and optionally further proteins of the profile are well-known and can be determined by routine techniques. This information is also readily available in various known databases, for example Uniprot or Expasy (prosite.expasy.org). Further information on some of the proteins and metabolites is provided in the Examples.

In various embodiments, in step a) the expression levels of not all components involved in the metabolic oxidative phosphorylation pathway are determined and provided to the mathematical model. This is due to fact that the gist of the invention lies in the finding that determination of ANT expression levels alone is sufficient to allow determining the oxidative phosphorylation profile of a cell sample with high accuracy (relative to determining the expression levels of all involved components). While under certain circumstances, accuracy may be further improved by including one or more additional targets the expression of which is determined and used in the described methods, it is generally desirable to keep the number of targets of which the protein/mRNA expression level needs to be determined as low as possible while maintaining high accuracy.

In other embodiments, the computer-implemented method comprises additionally quantitatively determining any one or more of the following individual metabolic parameters as input parameters that are to be provided to the mathematical model: heart rate, blood pressure, pressure-volume loops, and/or heart power, without being limited thereto.

In various embodiments, the method additionally comprises quantitatively determining metabolites in plasma, blood, or serum (e.g. peripheral, arterial or venous plasma or serum), preferably plasma, of said subject. The metabolites determined can be selected from, without limitation, glucose, lactate, pyruvate, glycerol, fatty acids, glutamate, glutamine, leucin, isoleucine, valine, acetate, beta-hydroxybutyrate, catecholamines, or insulin. In another embodiment, the metabolite can be determined in a tissue sample, e.g. a heart tissue sample. In another embodiment, the metabolite can be determined in a sample of urine, sweat or other body fluids. The metabolite concentration may vary over time.

In various embodiments, if the expression level of step a) is an mRNA expression level, ANT mRNA levels in the sample are determined using an RNA quantification method selected from the group of Reverse Transcription Polymerase Chain Reaction (RT-PCR), Quantitative Real-Time PCR (qRT-PCR), Northern Blotting, RNA-Seq (bulk or single-cell RNA Sequencing), Microarrays, in situ hybridization (ISH), Digital Droplet PCR (ddPCR), and LAMP (Loop-mediated Isothermal Amplification).

In such embodiments, the method may comprise the steps of: a1) solubilizing the sample, a2) extracting the RNA from the solubilized sample of step a) according to the RNA quantification method. a3) transferring said extracted RNAs from step b) to a device, preferably a NGS sequencer, of said RNA quantification method, and a4) identifying and quantifying the RNAs in said sample.

The present invention further relates to a computer program product configured to execute the computer-implemented method according to the invention on a computer.

It is to be understood that the above embodiments of the computer-implemented method are also applicable for the computer program product configured to execute said computer-implemented method, and *vice versa.*

The computer-implemented method can be adapted for many uses, e.g. in the field of metabolic research, oncology, neuroscience, cardiovascular research, stem cell research, aging research, immunology, infection biology, pharmacokinetics, toxicology, nutrition science, sports science, cancer immunotherapy, mitochondrial research, transplantation medicine, virology, biotechnology, microbiology, environmental research, drug discovery, development, precision therapy, drug safety, combination therapy, diagnostics, cell therapy, monitoring, and epidemiology, without being limited thereto. The respective uses and methods also form part of the present invention.

The present invention is further illustrated by the following non-limiting examples.

### Examples

### Algorithm

The used algorithm aggregates a large number of experimentally determined relationships (such as sequences of enzymatic reactions, enzyme regulation, enzymatic properties, etc.). It represents the sum of experimentally validated data.

The results of the algorithm have been validated using other (orthogonal) methods:
- Biochemical assays: (https://doi.org/10.1038/s41467-018-04720-9). A total of 177 parameters were measured and determined by the algorithm.
- Seahorse device (phenotypic measurement of glycolysis and OXPHOS rates). The results obtained from the algorithm were validated using this device as well.
- Congruence: Over 50 evaluation projects demonstrate that the measurements from the algorithm are congruent with phenotypic observations.

### Significance of the Respiratory Chain Complexes for the Accuracy of the OXPHOS Analysis

The importance of each complex was tested for the algorithm's reliability by permuting the input data (64 options). The results were sorted based on their correlation with "reality."

Calibration: The study assumes that the algorithm provides "the truth." To this end, data for all 6 complexes, i.e. including data for all gene/protein expression levels shown in Table 2, were provided, and the result was set to a correlation analysis value of 1.

Study Group: A total of 7 projects were analyzed, corresponding to 211 samples. The source of data is shown in Table 3 below. Samples from brain, muscle, heart, and immune cells were studied, originating from humans and mice. Diseases studied included cancers, ALS, obesity, heart failure, and Alzheimer's disease.

In all projects oxidative phosphorylation profiles were obtained using expression data for complexes I - VI of the respiratory chain individually as well as in all possible binary, ternary, quaternary and quinary combinations. Overall 62 different combinations of data for complexes I-VI were used. For the individual complexes, I, II, etc., the respective protein/gene expression levels shown in Table 2 were used as input data. For example, for complex II, the following four protein/gene expression levels were used: SDHA, SDHB, SDHC and SDHD (SDH2, SDH1, SDH3 and SDH4). The obtained results were compared to a reference where the profile has been determined based on expression data for all six complexes (see explanation of calibration above). Correlation with the reference was calculated for each tested combination.

It was found in all 7 projects that ANT alone would yield a result that closely aligns with "reality", i.e. the reference in which expression data from all complexes has been used. The results confirm that ANT is suitable as a surrogate marker for the entire respiratory chain.

**Table 3**

| **Project** | **GEO accession** | **Sample number** | **Species** | **Organ** | **Disease** | **Healthy control** |
|---|---|---|---|---|---|---|
| #1 | GSE234245 | 101 | human | brain | ALS | yes |
| #2 | GSE226901 | 18 | human | brain | Alzheimers | yes |
| #3 | GSE220258 | 6 | mouse | immune cells | cancer | yes |
| #4 | GSE154825 | 16 | human | brain | Mitochondrial encephalomvopathv | yes |
| #5 | GSE191165 | 12 | human | brain | medulloblastoma | yes |
| #6 | GSE199078 | 8 | mouse | heart | cardiomyopathy | yes |
| #7 | GSE244120 | 50 | human | muscle | obesity | yes |

| | | | | | | |
|---|---|---|---|---|---|---|
| GEO accession: ncbi.nlm.nih.gov/geo/ | | | | | | |

## Claims

1. Computer-implemented method for determining the oxidative phosphorylation profile of a cell sample, the method comprising
a) determining the expression level(s) of adenine nucleotide translocator (ANT) and optionally one or more further targets involved in the oxidative metabolic phosphorylation pathway in the cell sample;
b) providing the expression level data obtained in step a) to a mathematical model for metabolic profiling; and
c) determining the oxidative phosphorylation profile of the cell sample by calculation using said mathematical model for metabolic profiling.

2. The computer-implemented method of claim 1, wherein
(i) the cell sample is a single cell, cell suspension, organoid, membrane-bound particle or a tissue sample; and/or
(ii) the oxidative phosphorylation profile is determined at single-cell, bulk, or spatial scale.

3. The computer-implemented method of claim 1 or 2, wherein the cell sample is a mammalian cell sample.

4. The computer-implemented method of any one of claims 1 to 3, wherein the cell sample is a human cell sample.

5. The method of any one of claims 1 to 4, wherein the expression level is determined by determining the total mRNA level and/or protein level of ANT and all isoforms thereof in the sample.

6. The computer implemented method of any one of claims 1 to 5, wherein the method further comprises the step of comparing the determined oxidative phosphorylation profile of the cell sample to a reference profile.

7. The computer-implemented method of claim 6, wherein the reference profile is a healthy cell profile or a diseased cell profile.

8. The computer-implemented method of claim 6 or 7, wherein a difference between the sample profile and the reference profile is
(a) indicative for a disease or disorder that affects the oxidative phosphorylation profile of a cell;
(b) used to determine susceptibility to a specific treatment of a disease or disorder;
(c) used for risk stratification to develop a disease or disorder;
(d) used to monitor the progression or treatment of a disease or disorder;
(e) used to screen potential pharmaceutical actives for their pharmaceutical activity, safety, and/or metabolism;
(f) used to determine the age, nutritional status and/or overall health of a subject; and/or
(g) used to determine the inflammation status, infection status, hereditary disease status, epidemiologic status, environmental harm, or intoxication status of a subject.

9. The computer-implemented method of any one of the preceding claims, wherein the mathematical model is parameterized using experimentally measured parameters or database parameters.

10. The computer-implemented method of any one of the preceding claims, wherein the mathematical model for metabolic profiling is an algorithm for quantifying metabolic rates for at least one, preferably at least 5, more preferably at least 10 , even more preferably at least 15 and up to 25 central metabolic pathways, preferably selected from the following central metabolic pathways: (1) glycogen metabolism, (2) fructose metabolism, (3) galactose metabolism, (4) glycolysis, (5) gluconeogenesis, (6) oxidative pentose phosphate pathway, (7) non-oxidative pentose phosphate pathway, (8) fatty acid synthesis, (9) triglyceride synthesis, (10) synthesis and degradation of lipid droplets and synthesis of VLDL lipoprotein, (11) cholesterol synthesis, (12) tricarbonic acid (TCA) cycle, (13) respiratory chain and oxidative phosphorylation, (14) beta-oxidation of fatty acids, (15) urea cycle, (16) ethanol metabolism, (17) ketone body metabolism, (18) ammonia formation, (19) serine utilization, (20) alanine utilization, (21) branched chain amino acid metabolism, (22) branched-chain amino acid metabolism (BCAA), (23) glutamine metabolism, and (24) glutamate metabolism and (25) reactive oxygen species detoxification metabolism (ROS homeostasis).

11. The computer-implemented method of claim 10, wherein
(1) the algorithm is for quantifying the cellular energy metabolism by quantifying metabolic rates for respiratory chain and oxidative phosphorylation; and/or
(2) the algorithm uses up to 618 protein/RNA levels selected from those set forth in Table 1; and/or
(3) the algorithm is the algorithm disclosed at https://static-content.springer.com/esm/art%3A10.1038%2Fs41416-019-0659-3/MediaObjects/41416_2019_659_MOESM2_ESM.xml.

12. The computer-implemented method of any one of the preceding claims, wherein the method further comprises determining additional physicochemical input parameters and/or the expression level(s) of one or more further targets in the cell sample and providing the obtained data to the mathematical model.

13. The computer-implemented method of any one of the preceding claims, wherein the determination of the ANT and optionally one or more further target expression level(s) is carried out using any one or more of mass spectrometry, Western blot, immunohistochemistry (IHC), ELISA, Immuno-PCR, Proximity Ligation Assay (PLA), aptamer assay, X-ray crystallography, NMR spectroscopy, cryo electron microscopy, protein microarray, gel electrophoresis, fluorescence in situ hybridization, qPCR, Northern blot, RNA microarray, RNA sequencing, single-cell RNA sequencing (scRNA-Seq), digital droplet PCR, branched DNA assays, nanostring, ribonuclease protection assay, poly(A) tail length assay, cap analysis of gene expression (CAGE), spatial genomics or spatial proteomics assay, flow cytometry, image cytometry and mass cytometry (CyTOF).

14. The computer-implemented method of any one of the preceding claims, provided that in step a) the expression levels of not all components involved in the metabolic oxidative phosphorylation pathway are determined and provided to the mathematical model, preferably wherein only the ANT expression level is determined and provided to the mathematical model.

15. Computer program product configured to execute the computer-implemented method of any one of claims 1 to 14, preferably steps b) and c) or step c) thereof, on a computer.
